# EUROPEAN PATENT SPECIFICATION

(11) **EP 3 894 063 B1**
(45) Date of publication and mention of the grant of the patent: **10.01.2024**
(21) Application number: 20726437.5
(22) Date of filing: 19.05.2020
(51) Int. Cl.: B01J 13/14, A01N 25/28, A61K 8/11, A61K 9/50, C11D 3/50

(54) **POLY(ESTER UREA) MICROCAPSULES**
POLY(ESTERHARNSTOFF)-MIKROKAPSELN
MICROCAPSULES DE POLY(ESTER-URÉE)

(30) Priority: 21.05.2019 US 201962850667 P; 05.07.2019 EP 19184796
(43) Date of publication of application: 20.10.2021
(73) Proprietor: Firmenich SA, 1242 Satigny (CH)
(72) Inventor: MA, Ling, Cranbury, NJ 08512 (US); FENG, Jingyu, Plainsboro, NJ 08536 (US); OUALI, Lahoussine, 1242 Satigny (CH); JERRI, Huda, Plainsboro, New Jersey 08536 (US)
(74) Representative: Strych, Sebastian
(86) International application number: PCT/EP2020/063890
(87) International publication number: WO 2020/234263

(56) References cited:
- EP-A1- 3 170 552
- JP-A- H0 871 406
- JP-B2- 3 518 899
- US-A1- 2013 337 023

## Description

### Technical Field

The present invention relates to a new process for the preparation of poly(ester urea) based microcapsules. Poly(ester urea) based microcapsules are also an object of the invention. Perfuming compositions and consumer products comprising said microcapsules, in particular perfumed consumer products in the form of home care or personal care products, are also part of the invention.

### Background of the Invention

One of the problems faced by the perfumery industry lies in the relatively rapid loss of olfactive benefit provided by odoriferous compounds due to their volatility, particularly that of "top-notes". In order to tailor the release rates of volatiles, delivery systems such as microcapsules containing a perfume are needed to protect and later release the core payload when triggered. A key requirement from the industry regarding these systems is to survive suspension in challenging bases without physically dissociating or degrading. This is referred to as *stability* for the delivery system. For instance, fragranced personal and household cleansers containing high levels of aggressive surfactant detergents are very challenging for the stability of microcapsules.

Polyurea and polyurethane-based microcapsule slurry are widely used for example in perfumery industry as they provide a long lasting pleasant olfactory effect after their applications on different substrates. Those microcapsules have been widely disclosed in the prior art (see for example WO2007/004166 or EP 2300146 from the Applicant). JPH0871406A discloses a method to prepare core-shell microcapsules, wherein the shell comprises a reaction product of L-lysine amino ethyl ester with a carbodiimide modified diphenylmethane diisocyanate.

There is still a need to provide new microcapsules, while not compromising on the performance of the microcapsules, in particular in terms of stability in a challenging medium such as a consumer product base, as well as in delivering a good performance in terms of active ingredient delivery, e.g. olfactive performance in the case of perfuming ingredients.

The present invention is proposing a solution to the above-mentioned problem by providing new poly(ester urea) based microcapsules and a process for preparing said microcapsules.

It has now been surprisingly found that performing core-shell microcapsules encapsulating hydrophobic materials, wherein active ingredients are obtained by reacting at least one polyisocyanate having at least two isocyanate groups with a polyaminoester. The process of the invention therefore provides a solution to the above-mentioned problems as it allows preparing microcapsules with the desired stability in challenging bases.

A first object of the invention relates to a process for the preparation of a poly(ester urea) based core-shell microcapsule slurry comprising the following steps:
a) dissolving at least one polyisocyanate having at least two isocyanate groups in a hydrophobic material, preferably a perfume, to form an oil phase;
b) preparing a dispersing phase comprising a stabilizer, wherein the dispersing phase is not miscible with the oil phase;
c) adding the oil phase obtained in step a) into the dispersing phase to form a two-phases dispersion;
d) performing a curing step to form core-shell microcapsules in the form of a slurry, wherein a polyaminoester is added in step a) and/or in the two-phases dispersion of step c) and wherein the polyaminoester is obtained by a reaction between a polyol and an amino-acid.

A second object of the invention is a poly(ester urea) based core-shell microcapsule comprising:
- an oil-based core comprising a hydrophobic material, preferably comprising a perfume oil, and
- a shell comprising a reaction product between at least one polyisocyanate having at least two isocyanate groups and a polyaminoester, wherein the polyaminoester is obtained by a reaction between a polyol and an amino-acid.

Disclosed but not claimed is poly(ester urea) based core-shell microcapsule slurry comprising at least one microcapsule made of:
- an oil-based core comprising a hydrophobic material, preferably comprising a perfume oil, and
- a shell comprising a reaction product between at least one polyisocyanate having at least two isocyanate groups and a polyaminoester.

Disclosed but not claimed is a poly(ester urea) based core-shell microcapsule slurry obtainable by the process as defined above.

Disclosed but not claimed is a perfuming composition comprising the microcapsule slurry as defined above, wherein the core comprises a perfume.

Disclosed but not claimed is a consumer product comprising the microcapsule slurry or a perfuming composition as defined above.

### Drawings

**Figure 1****:** TGA measurements of microcapsules A.
**Figure 2****:** TGA measurements of microcapsules B.
**Figures 3-5****:** Olfactive performance of microcapsules according to the invention (microcapsules A) versus free oil.
**Figure 6****:** Olfactive performance of microcapsules according to the invention (microcapsules C and D).

### Detailed description of the invention

Unless stated otherwise, percentages (%) are meant to designate a percentage by weight of a composition.

By **"hydrophobic material",** it is meant any hydrophobic material - single material or a mixture of material - which forms a two-phase dispersion when mixed with water.

By **"ingredient",** it is meant a single compound or a combination of ingredients.

By **"perfume or flavour oil",** it is meant a single perfuming or flavouring compound or a mixture of several perfuming or flavouring compounds.

By **"consumer product"** or **"end-product"** it is meant a manufactured product ready to be distributed, sold and used by a consumer.

For the sake of clarity, by the expression "dispersion" in the present invention it is meant a system in which particles are dispersed in a continuous phase of a different composition and it specifically includes a suspension or an emulsion.

A **"microcapsule",** or the similar, in the present invention it is meant that core-shell microcapsules have a particle size distribution in the micron range (e.g. a mean diameter(D [4,3]) comprised between about 1 and 3000 microns) and comprise an external solid polymer-based shell and an internal continuous oil phase enclosed by the external shell.

By **"poly(ester urea)-based"** wall or shell, it is meant that the polymer comprises both urea linkages and ester linkages, urea linkages being produced by the amino groups of the polyaminoester crosslinker capable of further reacting with isocyanate groups during interfacial polymerization, ester linkages coming from the polyaminoester and being retained in the polymer backbone.

By **"polyaminoester"** or **"polyaminoester cross-linker"** which are used indifferently, it is meant a reaction product between a polyol and an amino-acid having preferably the following formula: 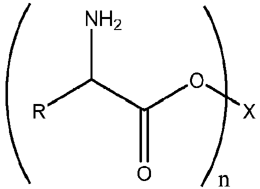
with n≥2, wherein R represents a hydrogen atom, a benzyl, a methyl, an *iso*-propyl, an *iso*-butyl or a *sec*-butyl group;
and wherein X is derivatived from a polyol of formula (HO)ₙ-X.

Preferably X is a C₁-C₁₀ hydrocarbon optionally substituted with 1 to 10 hydroxy groups or a - ((CH₂)ₚ-O)ₘ-(CH₂)ₚ group wherein p is an integer between 1 and 10 and m is an integer between 1 and 100. Preferably, X may be a C₁-C₁₀ alkane-di/tri/tetra/penta/hexa-yl group or a -((CH₂)ₚ-O)ₘ-(CH₂)ₚ group wherein p may be an integer between 1 and 5 and m may be an integer between 1 and 50. Even more preferably, X may be a C₁-C₅ alkane-di/tri/tetra-yl group or a - ((CH₂) ₚ-O)ₘ-(CH₂)ₚ group wherein p may be an integer between 1 and 3 and m may be an integer between 1 and 10. Even more preferably, X may be a C₁-C₅ alkane-di/tri/tetra-yl group or a -((CH₂) ₚ-O)ₘ-(CH₂)ₚ group wherein p may be an 2 and m may be an integer between 1 and 5.

According to any one embodiment n is an integer between 2 and 10. Preferably, n is an integer between 1 and 4.

The polyol has preferably the following formula: HO-((CH₂) ₚ-O)ₘ-(CH₂)ₚ-OH with n = 2 and with p and m being as defined above.

The polyaminoester is obtained by the reaction between a polyol and an amino-acid As non-limiting examples, the polyol can be chosen in the group consisting of glycerol, pentaerythritol, 1,1,1-tris(hydroxymethyl)ethane, 1,4-butanediol, diethylene glycol, and mixtures thereof.

As non-limiting examples, the amino-acid can be chosen in the group consisting of glycine, phenylalanine, alanine, valine, leucine, isoleucine and mixtures thereof.

According to a particular embodiment, the polyaminoester is chosen in the group consisting of the following compounds:

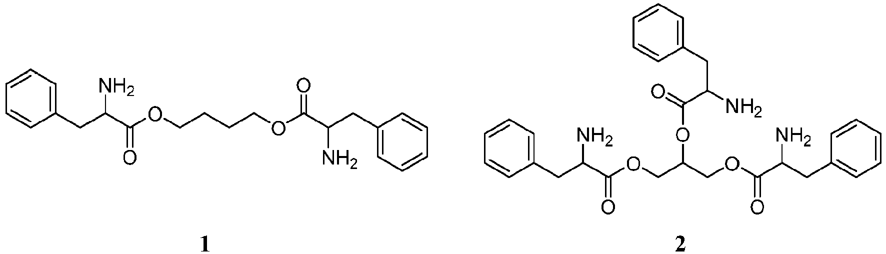

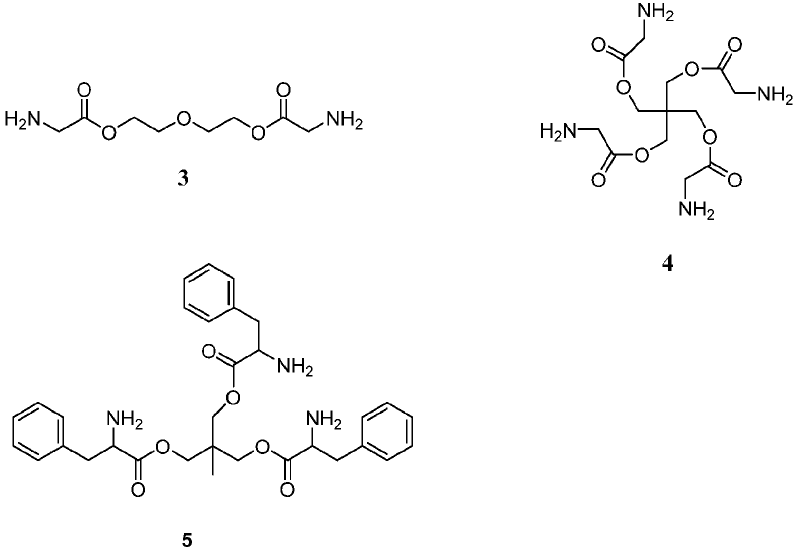

Thus, according to this embodiment, the polyaminoester is chosen in the group consisting of butane-1,4-diyl bis(2-amino-3-phenylpropanoate (**compound 1**), propane-1,2,3-triyl tris(2-amino-3-phenylpropanoate) (**compound 2**), oxybis(ethane-2,1-diyl) bis(2-aminoacetate) (**compound 3**), 2,2 bis((glycyloxy)methyl)propane-1,3-diyl bis(2-aminoacetate) (**compound 4**), [3-(2-amino-3-phenyl-propanoyl)oxy-2-[(2-amino-3-phenyl-propanoyl)oxymethyl]-2-methylpropyl] 2-amino-3-phenyl-propanoate (**compound 5**) and mixtures thereof.

Methods for preparing such compounds are well-known in the literature. One may cite for example, *Phenylalanine-Based Poly(ester urea): Synthesis, Characterization, and in vitro Degradation, Jiayi Yu, Fei Lin, Panpan Lin, Yaohua Gao, and Matthew L. Becker,*

*Macromolecules 2014, 47, 121-129* to prepare compound 1.

The synthesis of compound 1 can also be found in the literature: Versatile biodegradable poly(ester amide)s derived from alpha-amino acids for vascular tissue engineering, Karimi P, Rizkalla AS, Mequanint K., Materials 2010;3:2346-68*.*

Synthesis of compound 3 can be found in Novel Poly(ester amide)s From Glycine and L-lactic acid by an Easy and Cost-effective synthesis, Ana C. Fonseca et al. Polym Int 2013; 62: 736-743 *Page 23.*

The polyaminoester can be obtained in a salt form and then a base can be added (such as NaOH or Na₂CO₃) to deprotonate so as to obtain compound in its free base form.

It has been found that core-shell poly(ester urea)-based microcapsules with overall good performance in challenging bases could be obtained when at least one polyisocyanate having at least two isocyanate groups reacts with a polyaminoester.

### Process for preparing a poly(ester urea)-based microcapsule slurry

A first object of the invention is therefore a process for the preparation of a poly(ester urea) based core-shell microcapsule slurry comprising the following steps:
a) dissolving at least one polyisocyanate having at least two isocyanate groups in a hydrophobic material, preferably a perfume, to form an oil phase;
b) preparing a dispersing phase comprising a stabilizer, wherein the dispersing phase is not miscible with the oil phase;
c) adding the oil phase obtained in step a) into the dispersing phase to form a two-phases dispersion;
d) performing a curing step to form core-shell microcapsules in the form of a slurry, wherein a polyaminoester is added in step a) and/or in the two-phases dispersion of step c) and wherein the polyaminoester is obtained by a reaction between a polyol and an amino-acid.

According to an embodiment, the process comprises the following steps:
a) dissolving at least one polyisocyanate having at least two isocyanate groups and optionally a polyaminoester in a hydrophobic material, preferably a perfume, to form an oil phase;
b) preparing a dispersing phase comprising a stabilizer, wherein the dispersing phase is not miscible with the oil phase;
c) adding the oil phase obtained in step a) into the dispersing phase to form a two-phases dispersion;
d) adding a polyaminoester when not added in step a) to the dispersion obtained in step c); and
e) performing a curing step to form core-shell microcapsules in the form of a slurry, c) wherein the polyaminoester is obtained by a reaction between a polyol and an amino-acid.

According to an embodiment, in one step of the process, at least one polyisocyanate having at least two isocyanate groups and optionally a polyaminoester is dissolved in a hydrophobic material, preferably a perfume, to form an oil phase.

### Hydrophobic material

Hydrophobic material according to the invention can be "inert" material like solvents or active ingredients.

When, hydrophobic materials are active ingredient, it is preferably chosen from the group consisting of flavor, flavor ingredients, perfume, perfume ingredients, nutraceuticals, cosmetics, pest control agents, biocide actives and mixtures thereof.

According to a particular embodiment, the hydrophobic material comprises a mixture of a perfume with another ingredient selected from the group consisting of nutraceuticals, cosmetics, pest control agents and biocide actives.

According to a particular embodiment, the hydrophobic material comprises a mixture of biocide actives with another ingredient selected from the group consisting of perfume, nutraceuticals, cosmetics, pest control agents.

According to a particular embodiment, the hydrophobic material comprises a mixture of pest control agents with another ingredient selected from the group consisting of perfume, nutraceuticals, cosmetics, biocide actives.

According to a particular embodiment, the hydrophobic material comprises a perfume.

According to a particular embodiment, the hydrophobic material consists of a perfume.

According to a particular embodiment, the hydrophobic material consists of biocide actives.

According to a particular embodiment, the hydrophobic material consists of pest control agents.

By "perfume" (or also "perfume oil") what is meant here is an ingredient or composition that is a liquid at about 20°C. According to any one of the above embodiments said perfume oil can be a perfuming ingredient alone or a mixture of ingredients in the form of a perfuming composition. As a "perfuming ingredient" it is meant here a compound, which is used for the primary purpose of conferring or modulating an odour. In other words such an ingredient, to be considered as being a perfuming one, must be recognized by a person skilled in the art as being able to at least impart or modify in a positive or pleasant way the odor of a composition, and not just as having an odor. For the purpose of the present invention, perfume oil also includes combination of perfuming ingredients with substances which together improve, enhance or modify the delivery of the perfuming ingredients, such as perfume precursors, emulsions or dispersions, as well as combinations which impart an additional benefit beyond that of modifying or imparting an odor, such as long-lasting, blooming, malodour counteraction, antimicrobial effect, microbial stability, pest control.

The nature and type of the perfuming ingredients present in the oil phase do not warrant a more detailed description here, which in any case would not be exhaustive, the skilled person being able to select them on the basis of its general knowledge and according to intended use or application and the desired organoleptic effect. In general terms, these perfuming ingredients belong to chemical classes as varied as alcohols, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery.

In particular one may cite perfuming ingredients which are commonly used in perfume formulations, such as:
- Aldehydic ingredients: decanal, dodecanal, 2-methyl-undecanal, 10-undecenal, octanal, nonanal and/or nonenal;
- Aromatic-herbal ingredients: eucalyptus oil, camphor, eucalyptol, 5-methyltricyclo[6.2.1.0~2,7~]undecan-4-one, 1-methoxy-3-hexanethiol, 2-ethyl-4,4-dimethyl-1,3-oxathiane, 2,2,7/8,9/10-Tetramethylspiro[5.5]undec-8-en-1-one, menthol and/or alpha-pinene;

- Balsamic ingredients: coumarin, ethylvanillin and/or vanillin;
- Citrus ingredients: dihydromyrcenol, citral, orange oil, linalyl acetate, citronellyl nitrile, orange terpenes, limonene, 1-p-menthen-8-yl acetate and/or 1,4(8)-p-menthadiene;
   - Floral ingredients: methyl dihydrojasmonate, linalool, citronellol, phenyl ethanol, 3-(4-tert-butylphenyl)-2-methylpropanal, hexylcinnamic aldehyde, benzyl acetate, benzyl salicylate, tetrahydro-2-isobutyl-4-methyl-4(2H)-pyranol, beta ionone, methyl 2-(methylamino)benzoate, (E)-3-methyl-4-(2,6,6-trimethyl-2-cyclohexen-1-yl)-3-buten-2-one, (1E)-1-(2,6,6-trimethyl-2-cyclohexen-1-yl)-1-penten-3-one, 1-(2,6,6-trimethyl-1,3-cyclohexadien-1-yl)-2-buten-1-one, (2E)-1-(2,6,6-trimethyl-2-cyclohexen-1-yl)-2-buten-1-one, (2E)-1-[2,6,6-trimethyl-3-cyclohexen-1-yl] -2-buten-1-one, (2E)-1-(2,6,6-trimethyl-1-cyclohexen-1-yl)-2-buten-1-one, 2,5-dimethyl-2-indanmethanol, 2,6,6-trimethyl-3-cyclohexene-1-carboxylate, 3-(4,4-dimethyl-1-cyclohexen-1-yl)propanal, hexyl salicylate, 3,7-dimethyl-1,6-nonadien-3-ol, 3-(4-isopropylphenyl)-2-methylpropanal, verdyl acetate, geraniol, p-menth-1-en-8-ol, 4-(1,1-dimethylethyl)-1-cyclohexyle acetate, 1,1-dimethyl-2-phenylethyl acetate, 4-cyclohexyl-2-methyl-2-butanol, amyl salicylate , high cis methyl dihydrojasmonate, 3-methyl-5-phenyl-1-pentanol, verdyl proprionate, geranyl acetate, tetrahydro linalool, cis-7-p-menthanol, propyl (S)-2-(1,1-dimethylpropoxy)propanoate, 2-methoxynaphthalene, 2,2,2-trichloro-1-phenylethyl acetate, 4/3-(4-hydroxy-4-methylpentyl)-3-cyclohexene-1-carbaldehyde, amylcinnamic aldehyde, 8-decen-5-olide, 4-phenyl-2-butanone, isononyle acetate, 4-(1,1-dimethylethyl)-1-cyclohexyl acetate, verdyl isobutyrate and/or mixture of methylionones isomers;
   - Fruity ingredients: gamma-undecalactone, 2,2,5-trimethyl-5-pentylcyclopentanone, 2-methyl-4-propyl-1,3-oxathiane, 4-decanolide, ethyl 2-methyl-pentanoate, hexyl acetate, ethyl 2-methylbutanoate, gamma-nonalactone, allyl heptanoate, 2-phenoxyethyl isobutyrate, ethyl 2-methyl-1,3-dioxolane-2-acetate, 3-(3,3/1,1-dimethyl-5-indanyl)propanal, diethyl 1,4-cyclohexanedicarboxylate, 3-methyl-2-hexen-1-yl acetate, 1-[3,3-dimethylcyclohexyl]ethyl [3-ethyl-2-oxiranyl]acetate and/or diethyl 1,4-cyclohexane dicarboxylate;
   - Green ingredients: 2-methyl-3-hexanone (E)-oxime, 2,4-dimethyl-3-cyclohexene-1-carbaldehyde, 2-tert-butyl-1-cyclohexyl acetate, styrallyl acetate, allyl (2-methylbutoxy)acetate, 4-methyl-3-decen-5-ol, diphenyl ether, (Z)-3-hexen-1-ol and/or 1-(5,5-dimethyl-1-cyclohexen-1-yl)-4-penten-1-one;
   - Musk ingredients: 1,4-dioxa-5,17-cycloheptadecanedione, (Z)-4-cyclopentadecen-1-one, 3-methylcyclopentadecanone, 1-oxa-12-cyclohexadecen-2-one, 1-oxa-13-cyclohexadecen-2-one, (9Z)-9-cycloheptadecen-1-one, 2-{1S)-1-[(1R)-3,3-dimethylcyclohexyl]ethoxy}-2-oxoethyl propionate 3-methyl-5-cyclopentadecen-1-one, 1,3,4,6,7,8-hexahydro-4,6,6,7,8,8-hexamethyl-cyclopenta-g-2-benzopyrane, (1S,1'R)-2-[1-(3',3'-dimethyl-1'-cyclohexyl)ethoxy]-2-methylpropyl propanoate, oxacyclohexadecan-2-oneand/or (1S,1'R)-[1-(3',3'-dimethyl-1'-cyclohexyl)ethoxycarbonyl]methyl propanoate, ;
   - Woody ingredients: 1-[(1RS,6SR)-2,2,6-trimethylcyclohexyl]-3-hexanol, 3,3-dimethyl-5-[(1R)-2,2,3-trimethyl-3-cyclopenten-1-yl]-4-penten-2-ol, 3,4'-dimethylspiro[oxirane-2,9'-tricyclo[6.2.1.0^{2,7}]undec[4]ene, (1-ethoxyethoxy)cyclododecane, 2,2,9,11-tetramethylspiro[5.5]undec-8-en-1-yl acetate, 1-(octahydro-2,3,8,8-tetramethyl-2-naphtalenyl)-1-ethanone, patchouli oil, terpenes fractions of patchouli oil, clearwood^{®}, (1'R,E)-2-ethyl-4-(2',2',3'-trimethyl-3'-cyclopenten-1'-yl)-2-buten-1-ol, 2-ethyl-4-(2,2,3-trimethyl-3-cyclopenten-1-yl)-2-buten-1-ol, methyl cedryl ketone, 5-(2,2,3-trimethyl-3-cyclopentenyl)-3-methylpentan-2-ol, 1-(2, 3, 8, 8-tetramethyl-1, 2, 3,4, 6, 7, 8, 8 a-octahydronaphthalen-2-yl)ethan-1-one and/or isobornyl acetate;
   - Other ingredients (e.g. amber, powdery spicy or watery): dodecahydro-3a,6,6,9a-tetramethyl-naphtho[2,1-b]furan and any of its stereoisomers, heliotropin, anisic aldehyde, eugenol, cinnamic aldehyde, clove oil, 3-(1,3-benzodioxol-5-yl)-2-methylpropanal, 7-methyl-2H-1,5-benzodioxepin-3(4H)-one, 2,5,5-trimethyl-1,2,3,4,4a,5,6,7-octahydro-2-naphthalenol, 1-phenylvinyl acetate, 6-methyl-7-oxa-1-thia-4-azaspiro[4.4]nonan and/or 3-(3-isopropyl-1-phenyl)butanal.

It is also understood that perfuming ingredients may also be compounds known to release in a controlled manner various types of perfuming compounds also known as properfume or profragrance. Non-limiting examples of suitable properfume may include 4-(dodecylthio)-4-(2,6,6-trimethyl-2-cyclohexen-1-yl)-2-butanone, 4-(dodecylthio)-4-(2,6,6-trimethyl-1-cyclohexen-1-yl)-2-butanone, trans-3-(dodecylthio)-1-(2,6,6-trimethyl-3-cyclohexen-1-yl)-1-butanone, 2-phenylethyl oxo(phenyl)acetate or a mixture thereof.

According to a particular embodiment, the perfuming ingredients have a high steric hindrance and are chosen in particular in the following groups:
- Group 1: perfuming ingredients comprising a cyclohexane, cyclohexene, cyclohexanone or cyclohexenone ring substituted with at least one linear or branched C₁ to C₄ alkyl or alkenyl substituent;
- Group 2: perfuming ingredients comprising a cyclopentane, cyclopentene, cyclopentanone or cyclopentenone ring substituted with at least one linear or branched C₄ to C₈ alkyl or alkenyl substituent;
- Group 3: perfuming ingredients comprising a phenyl ring or perfuming ingredients comprising a cyclohexane, cyclohexene, cyclohexanone or cyclohexenone ring substituted with at least one linear or branched C₅ to C₈ alkyl or alkenyl substituent or with at least one phenyl substituent and optionally one or more linear or branched C₁ to C₃ alkyl or alkenyl substituents;
- Group 4: perfuming ingredients comprising at least two fused or linked C₅ and/or C₆ rings;
- Group 5: perfuming ingredients comprising a camphor-like ring structure;
- Group 6: perfuming ingredients comprising at least one C7 to C20 ring structure;
- Group 7: perfuming ingredients having a logP value above 3.5 and comprising at least one tert-butyl or at least one trichloromethyl substitutent;

Examples of ingredients from each of these groups are:
- Group 1: 2,4-dimethyl-3-cyclohexene-1-carbaldehyde (origin: Firmenich SA, Geneva, Switzerland), isocyclocitral, menthone, isomenthone, Romascone^{®} (methyl 2,2-dimethyl-6-methylene-1-cyclohexanecarboxylate, origin: Firmenich SA, Geneva, Switzerland), nerone, terpineol, dihydroterpineol, terpenyl acetate, dihydroterpenyl acetate, dipentene, eucalyptol, hexylate, rose oxide, Perycorolle^{®} ((S)-1,8-p-menthadiene-7-ol, origin: Firmenich SA, Geneva, Switzerland), 1-p-menthene-4-ol, (1RS,3RS,4SR)-3-p-mentanyl acetate, (1R,2S,4R)-4,6,6-trimethyl-bicyclo[3,1,1]heptan-2-ol, Doremox^{®} (tetrahydro-4-methyl-2-phenyl-2H-pyran, origin: Firmenich SA, Geneva, Switzerland), cyclohexyl acetate, cyclanol acetate, Fructalate^{®} (1,4-cyclohexane diethyldicarboxylate, origin: Firmenich SA, Geneva, Switzerland), Koumalactone^{®} ((3ARS,6SR,7ASR)-perhydro-3,6-dimethyl-benzo[B]furan-2-one, origin: Firmenich SA, Geneva, Switzerland), Natactone^{®} ((6R)-perhydro-3,6-dimethyl-benzo[B]furan-2-one, origin: Firmenich SA, Geneva, Switzerland), 2,4,6-trimethyl-4-phenyl-1,3-dioxane, 2,4,6-trimethyl-3-cyclohexene-1-carbaldehyde;
- Group 2: (E)-3-methyl-5-(2,2,3-trimethyl-3-cyclopenten-1-yl)-4-penten-2-ol (origin: Givaudan SA, Vernier, Switzerland), (1'R,E)-2-ethyl-4-(2',2',3'-trimethyl-3'-cyclopenten-1'-yl)-2-buten-1-ol (origin: Firmenich SA, Geneva, Switzerland), Polysantol^{®} ((1'R,E)-3,3-dimethyl-5-(2',2',3'-trimethyl-3'-cyclopenten-1'-yl)-4-penten-2-ol, origin: Firmenich SA, Geneva, Switzerland), fleuramone, Hedione^{®} HC (methyl-cis-3-oxo-2-pentyl-1-cyclopentane acetate, origin: Firmenich SA, Geneva, Switzerland), Veloutone^{®} (2,2,5-Trimethyl-5-pentyl-1-cyclopentanone, origin: Firmenich SA, Geneva, Switzerland), Nirvanol^{®} (3,3-dimethyl-5-(2,2,3-trimethyl-3-cyclopenten-1-yl)-4-penten-2-ol, origin: Firmenich SA, Geneva, Switzerland), 3-methyl-5-(2,2,3-trimethyl-3-cyclopenten-1-yl)-2-pentanol (origin, Givaudan SA, Vernier, Switzerland);
- Group 3: damascones, Neobutenone^{®} (1-(5,5-dimethyl-1-cyclohexen-1-yl)-4-penten-1-one, origin: Firmenich SA, Geneva, Switzerland), nectalactone ((1'R)-2-[2-(4'-methyl-3'-cyclohexen-1'-yl)propyl]cyclopentanone), alpha-ionone, beta-ionone, damascenone, Dynascone^{®} (mixture of 1-(5,5-dimethyl-1-cyclohexen-1-yl)-4-penten-1-one and 1-(3,3-dimethyl-1-cyclohexen-1-yl)-4-penten-1-one, origin: Firmenich SA, Geneva, Switzerland), Dorinone^{®} beta (1-(2,6,6-trimethyl-1-cyclohexen-1-yl)-2-buten-1-one, origin: Firmenich SA, Geneva, Switzerland), Romandolide^{®} ((1S,1'R)-[1-(3',3'-Dimethyl-1'-cyclohexyl)ethoxycarbonyl]methyl propanoate, origin: Firmenich SA, Geneva, Switzerland), 2-tert-butyl-1-cyclohexyl acetate (origin: International Flavors and Fragrances, USA), Limbanol^{®} (1-(2,2,3,6-tetramethyl-cyclohexyl)-3-hexanol, origin: Firmenich SA, Geneva, Switzerland), trans-1-(2,2,6-trimethyl-1-cyclohexyl)-3-hexanol (origin: Firmenich SA, Geneva, Switzerland), (E)-3-methyl-4-(2,6,6-trimethyl-2-cyclohexen-1-yl)-3-buten-2-one, terpenyl isobutyrate, Lorysia^{®} (4-(1,1-dimethylethyl)-1-cyclohexyl acetate, origin: Firmenich SA, Geneva, Switzerland), 8-methoxy-1-p-menthene, Helvetolide^{®} ((1S,1'R)-2-[1-(3',3'-dimethyl-1'-cyclohexyl) ethoxy]-2-methylpropyl propanoate, origin: Firmenich SA, Geneva, Switzerland), para tert-butylcyclohexanone, menthenethiol, 1-methyl-4-(4-methyl-3-pentenyl)-3-cyclohexene-1-carbaldehyde, allyl cyclohexylpropionate, cyclohexyl salicylate, 2-methoxy-4-methylphenyl methyl carbonate, ethyl 2-methoxy-4-methylphenyl carbonate, 4-ethyl-2-methoxyphenyl methyl carbonate;
- Group 4: Methyl cedryl ketone (origin: International Flavors and Fragrances, USA), Verdylate, vetyverol, vetyverone, 1-(octahydro-2,3,8,8-tetramethyl-2-naphtalenyl)-1-ethanone (origin: International Flavors and Fragrances, USA), (5RS,9RS,10SR)-2,6,9,10-tetramethyl-1-oxaspiro[4.5]deca-3,6-diene and the (5RS,9SR,10RS) isomer, 6-ethyl-2,10,10-trimethyl-1-oxaspiro[4.5]deca-3,6-diene, 1,2,3,5,6,7-hexahydro-1,1,2,3,3-pentamethyl-4-indenone (origin: International Flavors and Fragrances, USA), Hivernal^{®} (a mixture of 3-(3,3-dimethyl-5-indanyl)propanal and 3-(1,1-dimethyl-5-indanyl)propanal, origin: Firmenich SA, Geneva, Switzerland), Rhubofix^{®} (3',4-dimethyl-tricyclo[6.2.1.0(2,7)]undec-4-ene-9-spiro-2'-oxirane, origin: Firmenich SA, Geneva, Switzerland), 9/10-ethyldiene-3-oxatricyclo[6.2.1.0(2,7)]undecane, Polywood^{®} (perhydro-5,5,8A-trimethyl-2-naphthalenyl acetate, origin: Firmenich SA, Geneva, Switzerland), octalynol, Cetalox^{®} (dodecahydro-3a,6,6,9a-tetramethyl-naphtho[2,1-b]furan, origin: Firmenich SA, Geneva, Switzerland), tricyclo[5.2.1.0(2,6)]dec-3-en-8-yl acetate and tricyclo[5.2.1.0(2,6)]dec-4-en-8-yl acetate as well as tricyclo[5.2.1.0(2,6)]dec-3-en-8-yl propanoate and tricyclo[5.2.1.0(2,6)]dec-4-en-8-yl propanoate, (+)-(1S,2S,3S)-2,6,6-trimethyl-bicyclo[3.1.1]heptane-3-spiro-2'-cyclohexen-4'-one;
- Group 5: camphor, borneol, isobornyl acetate, 8-isopropyl-6-methyl-bicyclo[2.2.2]oct-5-ene-2-carbaldehyde, camphopinene, cedramber (8-methoxy-2,6,6,8-tetramethyl-tricyclo[5.3.1.0(1,5)]undecane, origin: Firmenich SA, Geneva, Switzerland), cedrene, cedrenol, cedrol, Florex^{®} (mixture of 9-ethylidene-3-oxatricyclo[6.2.1.0(2,7)]undecan-4-one and 10-ethylidene-3-oxatricyclo[6.2.1.0(2,7)]undecan-4-one, origin: Firmenich SA, Geneva, Switzerland), 3-methoxy-7,7-dimethyl-10-methylene-bicyclo[4.3.1]decane (origin: Firmenich SA, Geneva, Switzerland);
- Group 6: Cedroxyde^{®} (trimethyl-13-oxabicyclo-[10.1.0]-trideca-4,8-diene , origin: Firmenich SA, Geneva, Switzerland), Ambrettolide LG ((E)-9-hexadecen-16-olide, origin: Firmenich SA, Geneva, Switzerland), Habanolide^{®} (pentadecenolide, origin: Firmenich SA, Geneva, Switzerland), muscenone (3-methyl-(4/5)-cyclopentadecenone, origin: Firmenich SA, Geneva, Switzerland), muscone (origin: Firmenich SA, Geneva, Switzerland), Exaltolide^{®} (pentadecanolide, origin: Firmenich SA, Geneva, Switzerland), Exaltone^{®} (cyclopentadecanone, origin: Firmenich SA, Geneva, Switzerland), (1-ethoxyethoxy)cyclododecane (origin: Firmenich SA, Geneva, Switzerland), Astrotone, 4,8-cyclododecadien-1-one;
- Group 7: Lilial^{®} (origin: Givaudan SA, Vernier, Switzerland), rosinol.

According to an embodiment, the perfume comprises at least 30%, particularly at least 50%, more particularly at least 60% of ingredients selected from Groups 1 to 7, as defined above. According to an embodiment, said perfume comprises at least 30%, particularly at least 50% of ingredients from Groups 3 to 7, as defined above. According to an embodiment, said perfume comprises at least 30%, particularly at least 50% of ingredients from Groups 3, 4, 6 or 7, as defined above.

According to an embodiment, the perfume comprises at least 30%, particularly at least 50%, more particularly at least 60% of ingredients having a logP above 3, particularly above 3.5 and even more particularly above 3.75.

According to an embodiment, the perfume used in the invention contains less than 10% of its own weight of primary alcohols, less than 15% of its own weight of secondary alcohols and less than 20% of its own weight of tertiary alcohols. According to an embodiment, the perfume used in the invention does not contain any primary alcohols and contains less than 15% of secondary and tertiary alcohols.

The perfuming ingredients may be dissolved in a solvent of current use in the perfume industry. According to an embodiment, the solvent is not an alcohol. Examples of such solvents are diethyl phthalate, isopropyl myristate, Abalyn^{®} (rosin resins, available from Eastman), benzyl benzoate, ethyl citrate, limonene or other terpenes, or isoparaffins. According to an embodiment, the solvent is very hydrophobic and highly sterically hindered, like for example Abalyn^{®} or benzyl benzoate. According to an embodiment, the perfume comprises less than 30% of solvent. According to an embodiment, the perfume comprises less than 20% and even more particularly less than 10% of solvent, all these percentages being defined by weight relative to the total weight of the perfume. According to an embodiment, the perfume is essentially free of solvent.

According to an embodiment, the oil phase (or the oil-based core) comprises:
- 25-100wt% of a perfume oil comprising at least 15wt% of high impact perfume raw materials having a Log T<-4, and
- 0-75wt% of a density balancing material having a density greater than 1.07 g/cm³.
The nature of high impact perfume raw materials having a Log T<-4 and density balancing material having a density greater than 1.07 g/cm³ are described in WO2018115250, the content of which are included by reference.

According to a particular embodiment, the hydrophobic material is free of any active ingredient (such as perfume). According to this particular embodiment, it comprises, preferably consists of hydrophobic solvents, preferably chosen in the group consisting of isopropyl myristate, tryglycerides (e.g. Neobee^{®} MCT oil, vegetable oils), D-limonene, silicone oil, mineral oil, and mixtures thereofwith optionally hydrophilic solvents preferably chosen in the group consisting of1,4 butanediol, benzyl alcohol, triethyl citrate, triacetin, benzyl acetate, ethyl acetate, propylene glycol (1,2-propanediol), 1,3-Propanediol, dipropylene glycol, glycerol, glycol ethers and mixtures thereof.

According to any one of the invention's embodiments, the hydrophobic material represents between about 10% and 60% w/w, or even between 15% and 45% w/w, by weight, relative to the total weight of the dispersion as obtained after step c).

According to a particular embodiment, the oil phase essentially consists of the polyisocyanate, optionally the polyaminoester and a perfume or flavor oil.

The term "biocide" refers to a chemical substance capable of killing living organisms (e.g. microorganisms) or reducing or preventing their growth and/or accumulation. Biocides are commonly used in medicine, agriculture, forestry, and in industry where they prevent the fouling of, for example, water, agricultural products including seed, and oil pipelines. A biocide can be a pesticide, including a fungicide, herbicide, insecticide, algicide, molluscicide, miticide and rodenticide; and/or an antimicrobial such as a germicide, antibiotic, antibacterial, antiviral, antifungal, antiprotozoal and/or antiparasite.

As used herein, a "pest control agent" indicates a substance that serves to repel or attract pests, to decrease, inhibit or promote their growth, development or their activity. Pests refer to any living organism, whether animal, plant or fungus, which is invasive or troublesome to plants or animals, pests include insects notably arthropods, mites, spiders, fungi, weeds, bacteria and other microorganisms.

When a polyaminoester is also dissolved in the hydrophobic material in step a), it is preferably used as a free base.

### Polyisocyanate having at least two isocyanate groups

Said polyisocyanate may comprise up to 6, or even only 4 isocyanate functional groups. According to any of the above embodiments, said polyisocyanate contains at least three isocyanate functional groups.

Low volatility polyisocyanates are preferred because of their low toxicity.

The at least one polyisocyanate may be aliphatic, aromatic or a mixture of both aromatic and aliphatic polyisocyanates. In the case of mixtures of polyisocyanates, each member of the mixture has at least two isocyanate functional groups.

According to one embodiment, the at least one polyisocyanate is an aromatic polyisocyanate.

The term "aromatic polyisocyanate" is meant here as encompassing any polyisocyanate comprising an aromatic moiety. Preferably, it comprises a phenyl, a toluyl, a xylyl, a naphthyl or a diphenyl moiety, more preferably a toluyl or a xylyl moiety. Preferred aromatic polyisocyanates are biurets and polyisocyanurates, more preferably comprising one of the above-cited specific aromatic moieties. More preferably, the aromatic polyisocyanate is a polyisocyanurate of toluene diisocyanate (commercially available from Bayer under the tradename Desmodur^{®} RC), a trimethylol propane-adduct of toluene diisocyanate (commercially available from Bayer under the tradename Desmodur^{®} L75), a trimethylol propane-adduct of xylylene diisocyanate (commercially available from Mitsui Chemicals under the tradename Takenate^{®} D-110N). In a most preferred embodiment, the aromatic polyisocyanate is a trimethylol propane-adduct of xylylene diisocyanate.

According to another embodiment, said polyisocyanate is an aliphatic polyisocyanate.

The term "aliphatic polyisocyanate" is defined as a polyisocyanate which does not comprise any aromatic moiety. Preferred aliphatic polyisocyanates are a trimer of hexamethylene diisocyanate, a trimer of isophorone diisocyanate, a trimethylol propane-adduct of hexamethylene diisocyanate (available from Mitsui Chemicals) or a biuret of hexamethylene diisocyanate (commercially available from Bayer under the tradename Desmodur^{®} N 100), among which a biuret of hexamethylene diisocyanate is even more preferred.

According to another embodiment, said at least one polyisocyanate is in the form of a mixture of at least one aliphatic polyisocyanate and of at least one aromatic polyisocyanate, both comprising at least two or three isocyanate functional groups, such as a mixture of a biuret of hexamethylene diisocyanate with a trimethylol propane-adduct of xylylene diisocyanate, a mixture of a biuret of hexamethylene diisocyanate with a polyisocyanurate of toluene diisocyanate and a mixture of a biuret of hexamethylene diisocyanate with a trimethylol propane-adduct of toluene diisocyanate. Most preferably, it is a mixture of a biuret of hexamethylene diisocyanate with a trimethylol propane-adduct of xylylene diisocyanate.

In a preferred embodiment, the at least one aliphatic polyisocyanate and the at least one aromatic polyisocyanate are used in a respective molar ratio comprised between 80:20 and 10:90, preferably between 75:25 and 20:80, more preferably between 60:40 and 20:80, even more preferably between 60:40 and 30:70, most preferably between 45:55 and 30:70.

The at least one polyisocyanate used in the process according to the invention is present in amounts representing from 1 to 15%, preferably from 2 to 8% and more preferably from 2 to 6% by weight of the microcapsule slurry.

According to an embodiment, the polyisocyanate is not an amino-acid derived polyisocyanate having at least 2 isocyanate functional groups.

In another step of the process, a dispersing phase comprising a stabilizer is prepared.

### Dispersing phase

There is no restriction regarding the nature of the solvent that can be used in step b) as long as it can dissolve the stabilizer.

According to a particular embodiment, the dispersing phase comprises, preferably consists of water.

According to another particular embodiment, the content of water is below or equal to 10%, preferably below or equal to 5%, more preferably below or equal to 3% by weight based on the total weight of the dispersing phase.

According to a particular embodiment, the dispersing phase is free of water.

According to an embodiment, the dispersing phase comprises a solvent chosen in the group consisting of glycerol, 1,4-butanediol, ethylene glycol and mixtures thereof.

### Stabilizer

According to the invention, the stabilizer can be ionic or non-ionic.

According to the invention, the ionic stabilizer is chosen in the group consisting of gum Arabic, carboxymethyl cellulose, soy protein, sodium caseinate, gelatin, bovine serum albumin, sugar beet pectin, hydrolyzed soy protein, hydrolyzed sericin, Pseudocollagen, Biopolymer SA-N (INCI name : Hyaluronic Acid (and) Serum Albumen (and) Dextran Sulfate), Pentacare-NA PF (Hydrolyzed Wheat Gluten (and) Ceratonia Siliqua (Carob) Gum (and) Aqua (and) Sodium Dextran Sulfate (and) Bis-Hydroxyethyl Tromethamine (and) Phenoxyethanol (and) Ethylhexylglycerin), and mixtures thereof.

According to a preferred embodiment, the ionic stabilizer is chosen in the group consisting of gum Arabic, carboxymethyl cellulose, sodium caseinate, sugar beet pectin and mixtures thereof.

According to the invention, the non-ionic emulsifier is chosen in the group consisting of polyvinyl alcohol, modified polyvinyl alcohol, modified starch, modified cellulose, polysaccharides, and mixtures thereof.

According to a particular embodiment, the non-ionic emulsifier is chosen in the group consisting of polyvinyl alcohol, modified starch and mixtures thereof.

According to a particular embodiment, the stabilizer is chosen in the group consisting of gum Arabic, modified starch, polyvinyl alcohol, polyvinylpyrolidone (PVP), carboxymethylcellulose (CMC), anionic polysaccharides, acrylamide copolymer, inorganic particles, protein such as soy protein, rice protein, whey protein, white egg albumin, sodium caseinate, gelatin, bovine serum albumin, hydrolyzed soy protein, hydrolyzed sericin, pseudocollagen, silk protein, sericin powder, and mixtures thereof.

According to any one of the above embodiments of the present invention, the emulsion comprises between about 0.1% and 5% w/w of at least a stabilizer, percentage being expressed on a w/w basis relative to the total weight of the dispersion as obtained after step c). In still another aspect of the invention, the emulsion comprises between about 0.1% and 2% w/w of at least a stabilizer. In still another aspect of the invention, the emulsion comprises between about 0.1% and 1% w/w of at least a stabilizer.

According to an embodiment, in another step of the process, a polyaminoester as defined above is added to the two-phases dispersion obtained in step c), when said polyaminoester is not added in step a).

According to a particular embodiment, the process comprises the following steps:
a) dissolving at least one polyisocyanate having at least two isocyanate groups in a hydrophobic material, preferably a perfume, to form an oil phase;
b) preparing a dispersing phase comprising a stabilizer, wherein the dispersing phase is not miscible with the oil phase;
c) adding the oil phase obtained in step a) into the dispersing phase to form a two-phases dispersion;
d) adding a polyaminoester to the dispersion obtained in step c); and
e) performing a curing step to form core-shell microcapsules in the form of a slurry, c) and wherein the polyaminoester is obtained by a reaction between a polyol and an amino-acid.

The polyaminoester of the invention is as described previously. The polyaminoester is preferably used an amount of between 0.1 and 15%, more preferably between 4 and 12%, these percentages being defined by weight relative to the total weight of the slurry.

According to a particular embodiment, the molar ratio between the amino groups of the polyaminoester and the isocyanate groups is comprised between 0.1 and 3, preferably between 0.5 and 1.

This is followed by a curing step d) or e) which allows ending up with microcapsules in the form of a slurry. The curing step consists of reacting the polyaminoester and the polyisocyanate in a sufficient time to form microcapsules in the form of a slurry.

According to a preferred embodiment, to enhance the kinetics, said step is performed at a temperature comprised between 60 and 80°C, possibly under pressure, for 1 to 4 hours. More preferably it is performed at between 50 and 90°C for between 30 minutes and 4 hours. However, the curing step can take place at room temperature.

### Optional outer coating

According to a particular embodiment of the invention, at the end of step d) or e) or during step d) or e), one may also add to the invention's slurry a polymer selected from the group consisting of a non-ionic polysaccharide, a cationic polymer and mixtures thereof to form an outer coating to the microcapsule.

Non-ionic polysaccharide polymers are well known to a person skilled in the art and are described for instance in WO2012/007438 page 29, lines 1 to 25 and in WO2013/026657 page 2, lines 12 to 19 and page 4, lines 3 to 12. Preferred non-ionic polysaccharides are selected from the group consisting of locust bean gum, xyloglucan, guar gum, hydroxypropyl guar, hydroxypropyl cellulose and hydroxypropyl methyl cellulose.

Cationic polymers are well known to a person skilled in the art. Preferred cationic polymers have cationic charge densities of at least 0.5 meq/g, more preferably at least about 1.5 meq/g, but also preferably less than about 7 meq/g, more preferably less than about 6.2 meq/g. The cationic charge density of the cationic polymers may be determined by the Kjeldahl method as described in the US Pharmacopoeia under chemical tests for Nitrogen determination. The preferred cationic polymers are chosen from those that contain units comprising primary, secondary, tertiary and/or quaternary amine groups that can either form part of the main polymer chain or can be borne by a side substituent directly connected thereto. The weight average (Mw) molecular weight of the cationic polymer is preferably between 10,000 and 3.5M Dalton, more preferably between 50,000 and 1.5M Dalton. According to a particular embodiment, one will use cationic polymers based on acrylamide, methacrylamide, N-vinylpyrrolidone, quaternized N,N-dimethylaminomethacrylate, diallyldimethylammonium chloride, quaternized vinylimidazole (3-methyl-1-vinyl-1H-imidazol-3-ium chloride), vinylpyrrolidone, acrylamidopropyltrimonium chloride, cassia hydroxypropyltrimonium chloride, guar hydroxypropyltrimonium chloride or polygalactomannan 2-hydroxypropyltrimethylammonium chloride ether, starch hydroxypropyltrimonium chloride and cellulose hydroxypropyltrimonium chloride. Preferably copolymers shall be selected from the group consisting of polyquaternium-5, polyquaternium-6, polyquaternium-7, polyquaternium10, polyquaternium-11, polyquaternium-16, polyquaternium-22, polyquaternium-28, polyquaternium-43, polyquaternium-44, polyquaternium-46, cassia hydroxypropyltrimonium chloride, guar hydroxypropyltrimonium chloride or polygalactomannan 2-hydroxypropyltrimethylammonium chloride ether, starch hydroxypropyltrimonium chloride and cellulose hydroxypropyltrimonium chloride. As specific examples of commercially available products, one may cite Salcare^{®} SC60 (cationic copolymer of acrylamidopropyltrimonium chloride and acrylamide, origin: BASF) or Luviquat^{®}, such as the PQ 11N, FC 550 or Style (polyquaternium-11 to 68 or quaternized copolymers of vinylpyrrolidone origin: BASF), or also the Jaguar^{®} (C13S or C17, origin Rhodia).

According to any one of the above embodiments of the invention, there is added an amount of polymer described above comprised between about 0% and 5% w/w, or even between about 0.1% and 2% w/w, percentage being expressed on a w/w basis relative to the total weight of the slurry as obtained after step e). It is clearly understood by a person skilled in the art that only part of said added polymers will be incorporated into/deposited on the microcapsule shell.

Another object of the invention is a process for preparing a microcapsule powder comprising the steps as defined above and an additional step f) consisting of submitting the slurry obtained in step e) to a drying, like spray-drying, to provide the microcapsules as such, i.e. in a powdery form. It is understood that any standard method known by a person skilled in the art to perform such drying is also applicable. In particular the slurry may be spray-dried preferably in the presence of a polymeric carrier material such as polyvinyl acetate, polyvinyl alcohol, dextrins, natural or modified starch, vegetable gums, pectins, xanthans, alginates, carragenans or cellulose derivatives to provide microcapsules in a powder form.

However, one may cite also other drying method such as the extrusion, plating, spray granulation, the fluidized bed, or even a drying at room temperature using materials (carrier, desiccant) that meet specific criteria as disclosed in WO2017/134179.

According to a particular embodiment, the carrier material contains free perfume oil which can be the same or different from the perfume from the core of the microcapsules.

Another object of the disclosure is a poly(ester urea) microcapsule slurry obtainable by the process as described above.

### Poly(ester urea) microcapsule

The composition of the poly(ester urea) shell enables to provide microcapsules that show the desired stability in the product base (e.g. counteracts efficiently the extraction of the perfume by the surfactants of the consumer product).

Thus, another object of the invention is a poly(ester urea) core-shell microcapsule comprising:
- an oil-based core comprising a hydrophobic material, preferably comprising a perfume oil, and
- a shell comprising a reaction product between at least one polyisocyanate having at least two isocyanate groups and a polyaminoester, wherein the polyaminoester is obtained by a reaction between a polyol and an amino-acid.

Disclosed but not claimed is a poly(ester urea) core-shell microcapsule slurry comprising at least one microcapsule made of:
- an oil-based core comprising a hydrophobic material, preferably comprising a perfume oil, and
- a shell comprising a reaction product between at least one polyisocyanate having at least two isocyanate groups and a polyaminoester.

Another object of the invention is a solid particle comprising:
- a polymeric carrier material, preferably chosen in the group consisting of polyvinyl acetate, polyvinyl alcohol, dextrins, natural or modified starch, vegetable gums, pectins, xanthans, alginates, carragenans, cellulose derivatives and mixtures thereof, and
- microcapsules as defined above entrapped in said carrier material, and
- optionally free perfume entrapped in said carrier material.

Solid particle as defined above and microcapsule powder are used indifferently in the present invention.

The embodiments described previously, notably regarding the nature of the hydrophobic material, the colloidal stabilizer, the polyisocyanate, the polyaminoester also apply for the poly(ester urea) microcapsules.

### Perfuming composition/consumer products

The microcapsules of the invention can be used in combination with active ingredients. An object of the invention is therefore a composition comprising:
(i) microcapsules as defined above;
(ii) an active ingredient, preferably chosen in the group consisting of a cosmetic ingredient, skin caring ingredient, perfume ingredient, flavor ingredient, malodour counteracting ingredient, pharmaceutical or agrochemical ingredient, a sanitizing ingredient, an insect repellent or attractant, and mixtures thereof.

The capsules of the invention show a good performance in terms of stability in challenging medium.

Another object of the present invention is a perfuming composition comprising:
(i) microcapsules as defined above, wherein the oil comprises a perfume;
(ii) at least one ingredient selected from the group consisting of a perfumery carrier, a perfumery co-ingredient and mixtures thereof;
(iii) optionally at least one perfumery adjuvant.

As liquid perfumery carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples solvents such as dipropyleneglycol, diethyl phthalate, isopropyl myristate, benzyl benzoate, 2-(2-ethoxyethoxy)-1-ethanol or ethyl citrate, which are the most commonly used. For the compositions which comprise both a perfumery carrier and a perfumery co-ingredient, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company). By "perfumery co-ingredient" it is meant here a compound, which is used in a perfuming preparation or a composition to impart a hedonic effect and which is not a microcapsule as defined above. In other words such a co-ingredient, to be considered as being a perfuming one, must be recognized by a person skilled in the art as being able to at least impart or modify in a positive or pleasant way the odor of a composition, and not just as having an odor.

The nature and type of the perfuming co-ingredients present in the perfuming composition do not warrant a more detailed description here, which in any case would not be exhaustive, the skilled person being able to select them on the basis of his general knowledge and according to the intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds.

By "perfumery adjuvant" we mean here an ingredient capable of imparting additional added benefit such as a color, a particular light resistance, chemical stability, etc. A detailed description of the nature and type of adjuvant commonly used in perfuming bases cannot be exhaustive, but it has to be mentioned that said ingredients are well known to a person skilled in the art.

Preferably, the perfuming composition according to the invention comprises between 0.01 and 30 % by weight of microcapsules as defined above.

The invention's microcapsules can advantageously be used in many application fields and used in consumer products. Microcapsules can be used in liquid form applicable to liquid consumer products as well as in powder form, applicable to powder consumer products.

According to a particular embodiment, the consumer product as defined above is liquid and comprises:
a) from 2 to 65% by weight, relative to the total weight of the consumer product, of at least one surfactant;
b) water or a water-miscible hydrophilic organic solvent; and
c) microcapsule as defined above,
d) optionally non-encapsulated perfume.

According to a particular embodiment, the consumer product as defined above is in a powder form and comprises:
a) from 2 to 65% by weight, relative to the total weight of the consumer product, of at least one surfactant;
b) microcapsule powder as defined above.
c) optionally perfume powder that is different from the microcapsules defined above.

In the case of microcapsules including a perfume oil-based core, the products of the invention, can in particular be of used in perfumed consumer products such as product belonging to fine fragrance or "functional" perfumery. Functional perfumery includes in particular personal-care products including hair-care, body cleansing, skin care, hygiene-care as well as home-care products including laundry care and air care. Consequently, another object of the present invention consists of a perfumed consumer product comprising as a perfuming ingredient, the microcapsules defined above or a perfuming composition as defined above. The perfume element of said consumer product can be a combination of perfume microcapsules as defined above and free or non-encapsulated perfume, as well as other types of perfume microcapsule than those here-disclosed.

In particular a liquid consumer product comprising:
a) from 2 to 65% by weight, relative to the total weight of the consumer product, of at least one surfactant;
b) water or a water-miscible hydrophilic organic solvent; and
c) a perfuming composition as defined above is another object of the invention.

Also a powder consumer product comprising
(a) from 2 to 65% by weight, relative to the total weight of the consumer product, of at least one surfactant; and
(b) a perfuming composition as defined above is part of the invention.

The invention's microcapsules can therefore be added as such or as part of an invention's perfuming composition in a perfumed consumer product.

For the sake of clarity, it has to be mentioned that, by "perfumed consumer product" it is meant a consumer product which is expected to deliver among different benefits a perfuming effect to the surface to which it is applied (e.g. skin, hair, textile, paper, or home surface) or in the air (air-freshener, deodorizer etc). In other words, a perfumed consumer product according to the invention is a manufactured product which comprises a functional formulation also referred to as "base", together with benefit agents, among which an effective amount of microcapsules according to the invention.

The nature and type of the other constituents of the perfumed consumer product do not warrant a more detailed description here, which in any case would not be exhaustive, the skilled person being able to select them on the basis of his general knowledge and according to the nature and the desired effect of said product. Base formulations of consumer products in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.

Non-limiting examples of suitable perfumed consumer product can be a perfume, such as a fine perfume, a cologne, an after-shave lotion, a body-splash; a fabric care product, such as a liquid or solid detergent, tablets and pods, a fabric softener, a dryer sheet, a fabric refresher, an ironing water, or a bleach; a personal-care product, such as a hair-care product (e.g. a shampoo, hair conditioner, a colouring preparation or a hair spray), a cosmetic preparation (e.g. a vanishing cream, body lotion or a deodorant or antiperspirant), or a skin-care product (e.g. a perfumed soap, shower or bath mousse, body wash, oil or gel, bath salts, or a hygiene product); an air care product, such as an air freshener or a "ready to use" powdered air freshener; or a home care product, such all-purpose cleaners, liquid or power or tablet dishwashing products, toilet cleaners or products for cleaning various surfaces, for example sprays & wipes intended for the treatment / refreshment of textiles or hard surfaces (floors, tiles, stone-floors etc.); a hygiene product such as sanitary napkins, diapers, toilet paper.

Another object of the invention is a consumer product comprising:
- a personal care active base, and
- microcapsules as defined above or the perfuming composition as defined above,
wherein the consumer product is in the form of a personal care composition.

Personal care active base in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.

The personal care composition is preferably chosen in the group consisting of a hair-care product (e.g. a shampoo, hair conditioner, a colouring preparation or a hair spray), a cosmetic preparation (e.g. a vanishing cream, body lotion or a deodorant or antiperspirant), or a skin-care product (e.g. a perfumed soap, shower or bath mousse, body wash, oil or gel, bath salts, or a hygiene product);

Another object of the invention is a consumer product comprising:
- a home care or a fabric care active base, and
- microcapsules as defined above or the perfuming composition as defined above,
wherein the consumer product is in the form of a home care or a fabric care composition.

Home care or fabric care active base in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.

Preferably, the consumer product comprises from 0.1 to 15 wt%, more preferably between 0.2 and 5 wt% of the microcapsules of the present invention, these percentages being defined by weight relative to the total weight of the consumer product. Of course the above concentrations may be adapted according to the benefit effect desired in each product.

According to a particular embodiment, the consumer product is in the form of a fabric softener composition and comprises:
- between 85 and 99.9% of a fabric softener active base;
- between 0.1 to 15 wt%, more preferably between 0.2 and 5 wt% by weight of the microcapsules of the invention.

The fabric softener active base may comprise cationic surfactants of quaternary ammonium, such as Diethyl ester dimethyl ammonium chloride (DEEDMAC), TEAQ (triethanolamine quat), HEQ (Hamburg esterquat) and mixtures thereof.

An object of the invention is a consumer product in the form of a fabric softener composition comprising:
- a fabric softener active base; preferably chosen in the group consisting of dialkyl quaternary ammonium salts, dialkyl ester quaternary ammonium salts (esterquats), Hamburg esterquat (HEQ), TEAQ (triethanolamine quat), silicones, cationic guars and mixtures thereof, preferably in an amount comprised between 85 and 99.95% by weight based on the total weight of the composition,
- microcapsules as defined above, preferably in an amount comprised between 0.05 to 15 wt%, more preferably between 0.1 and 5 wt% by weight based on the total weight of the composition.

An object of the invention is a consumer product in the form of a liquid detergent composition comprising:
- a liquid detergent active base; preferably chosen in the group consisting of anionic surfactant such as alkylbenzenesulfonate (ABS), secondary alkyl sulfonate (SAS), primary alcohol sulfate (PAS), lauryl ether sulfate (LES), methyl ester sulfonate (MES) and nonionic surfactant such as alkyl amines, alkanolamide, fatty alcohol poly(ethylene glycol) ether, fatty alcohol ethoxylate (FAE), ethylene oxide (EO) and propylene oxide (PO) copolymers, amine oxydes, alkyl polyglucosides, alkyl polyglucosamides, preferably in an amount comprised between 85 and 99.95% by weight based on the total weight of the composition,
- the microcapsules as defined above, preferably in an amount comprised between 0.05 to 15 wt%, more preferably between 0.1 and 5 wt% by weight based on the total weight of the composition.

An object of the invention is a consumer product in the form of a solid detergent composition comprising:
- a solid detergent active base; preferably chosen in the group consisting of anionic surfactant such as alkylbenzenesulfonate (ABS), secondary alkyl sulfonate (SAS), primary alcohol sulfate (PAS), lauryl ether sulfate (LES), methyl ester sulfonate (MES) and nonionic surfactant such as alkyl amines, alkanolamide, fatty alcohol poly(ethylene glycol) ether, fatty alcohol ethoxylate (FAE), ethylene oxide (EO) and propylene oxide (PO) copolymers, amine oxydes, alkyl polyglucosides, alkyl polyglucosamides, preferably in an amount comprised between 85 and 99.95% by weight based on the total weight of the composition,
- the microcapsules as defined above, preferably in an amount comprised between 0.05 to 15 wt%, more preferably between 0.1 and 5 wt% by weight based on the total weight of the composition.

An object of the invention is a consumer product in the form of a solid scent booster comprising:
- a solid carrier, preferably chosen in the group consisting of urea, sodium chloride, sodium sulphate, sodium acetate, zeolite, sodium carbonate, sodium bicarbonate, clay, talc, calcium carbonate, magnesium sulfate, gypsum, calcium sulfate, magnesium oxide, zinc oxide, titanium dioxide, calcium chloride, potassium chloride, magnesium chloride, zinc chloride, saccharides such as sucrose, mono-, di-, and polysaccharides and derivatives such as starch, cellulose, methyl cellulose, ethyl cellulose, propyl cellulose, polyols/sugar alcohols such as sorbitol, maltitol, xylitol, erythritol, and isomalt, PEG, PVP, citric acid or any water soluble solid acid, fatty alcohols or fatty acids and mixtures thereof.
- the microcapsules as defined above, in a powdered form, preferably in an amount comprised between 0.05 to 15 wt%, more preferably between 0.1 and 5 wt% by weight based on the total weight of the composition.

An object of the invention is a consumer product in the form of a liquid scent booster comprising:
- an aqueous phase,
- a surfactant system essentially consisting of one or more than one non-ionic surfactant, wherein the surfactant system has a mean HLB between 10 and 14, preferably chosen in the group consisting of ethoxylated aliphatic alcohols, POE/PPG (polyoxyethylene and polyoxypropylene) ethers, mono and polyglyceryl esters, sucrose ester compounds, polyoxyethylene hydroxylesters, alkyl polyglucosides, amine oxides and combinations thereof;
- a linker chosen in the group consisting of alcohols, salts and esters of carboxylic acids, salts and esters of hydroxyl carboxylic acids, fatty acids, fatty acid salts, glycerol fatty acids, surfactant having an HLB less than 10 and mixtures thereof, and

- the microcapsules as defined above, preferably in an amount comprised between 0.05 to 15 wt%, more preferably between 0.1 and 5 wt% by weight based on the total weight of the composition.

An object of the invention is a consumer product in the form of a shampoo or a shower gel composition comprising:
- a shampoo or a shower gel active base; preferably chosen in the group consisting of sodium alkylether sulfate, ammonium alkylether sulfates, alkylamphoacetate, cocamidopropyl betaine, cocamide MEA, alkylglucosides and aminoacid based surfactants and mixtures thereof, preferably in an amount comprised between 85 and 99.95% by weight based on the total weight of the composition,
- the microcapsules as defined above, preferably in an amount comprised between 0.05 to 15 wt%, more preferably between 0.1 and 5 wt% by weight based on the total weight of the composition.

An object of the invention is a consumer product in the form of a rinse-off conditioner composition comprising:
- a rinse-off conditioner active base; preferably chosen in the group consisting of cetyltrimonium chloride, stearyl trimonium chloride, benzalkonium chloride, behentrimonium chloride and mixture thereof, preferably in an amount comprised between 85 and 99.95% by weight based on the total weight of the composition,
- the microcapsules as defined above, preferably in an amount comprised between 0.05 to 15 wt%, more preferably between 0.1 and 5 wt% by weight based on the total weight of the composition.

According to a particular embodiment, the consumer product is in the form of a perfuming composition comprising:
- 0.1 to 30%, preferably 0.1 to 20% of the microcapsules as defined above,
- 0 to 40%, preferably 3-40% of perfume, and
- 20-90, preferably 40-90% of ethanol, by weight based on the total weight of the perfuming composition.

The invention will now be further described by way of examples. It will be appreciated that the invention as claimed is not intended to be limited in any way by these examples.

### Examples

### Example 1

### Preparation of poly(ester urea) based microcapsules according to the invention

### Preparation of the polyaminoester crosslinker: butane-1,4-diyl bis (2-amino-3-phenylpropanoate)

The di-ammonium salt of L-phenylalanine and 1,4-butanediol diester p-toluene sulfonate was synthesized as follows.

In a 1000mL one neck round bottom flask equipped with a Dean-Stark apparatus and a magnetic stirrer toluene (200mL) was added followed by amino acids, L-Phenylalanine, (20g, 121mmol, 2.2eq. 165.19g/mol), p-toluenesulfonic acid monohydrate as catalyst (25.11g, 132 mmol, 2.4eq. 190.22g/mol), and 1,4 Butanediol (5g, 55 mmol, 1eq. 90.12g/mol). The reaction mixture was refluxed in oil bath for about 24hours (130°C) until no more water was produced. After it was cooled down to room temperature the toluene was evaporated under reduced pressure in the rotary evaporator. The resulting L-Phe based diesters were recrystallized from 200mL hot water. The melting point of the pure compound is 235°C as reported in the literature.

To prepare the aqueous solution of compound 1, the p-TSA salt of Phenylalanine (Phe) based diamine is added into water followed by adding base (NaOH or Na₂CO₃) to deprotonate to obtain compound 1 (butane-1,4-diyl bis (2-amino-3-phenylpropanoate).

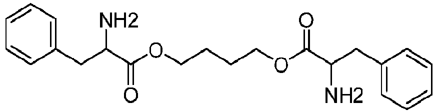

### Preparation of polyester (urea-urethane) microcapsules

**Table 1: Composition of capsules A according to the invention**

| **Ingredient** | **Percentage (w/w)** |
|---|---|
| Perfume Oil ¹⁾ | 31.05% |
| Takenate^{®} D-110N ²⁾ | 3.96% |
| Compound 1 | 11.38% |
| Water | 47.67% |
| Na₂CO₃ | 5.17% |
| PVOH | 0.77% |

| | |
|---|---|
| 1) Perfuming composition (perfume oil A) described in Table 2. 2) trimethylol propane adduct of xylylene diisocyanate; origin: Mitsui Chemicals, 75% polyisocyanate /25% ethyl acetate | |

**Table 2: Perfume oil A composition**

| **Ingredient** | **Concentration (Weight %)** |
|---|---|
| methyl 2,2-dimethyl-6-methylene-1-cyclohexanecarboxylate ¹⁾ | 20 |
| ((+-)-3-(4-isopro(2Zpylphenyl)-2-methylpropanal ²⁾ | 20 |
| 2-tert-butyl-1-cyclohexyl acetate ³⁾ | 20 |
| 4-tert-butyl-1-cyclohexyl acetate ⁴) | 20 |
| ((2Z)-2-phenyl-2-hexenenitrile ⁵⁾ | 20 |

| | |
|---|---|
| 1) Origin: Firmenich SA, Geneva, Switzerland; 2) Origin: Firmenich SA, Geneva, Switzerland; 3) Origin: International Flavors and Fragrances, USA; 4) Origin: International Flavors and Fragrances, USA; 5) Origin: Firmenich SA, Geneva, Switzerland | |

### General protocol for capsule synthesis:

At least one polyisocyanate (e.g. Takenate^{®} D-110N) was dissolved in a perfume oil. The oil phase was then added to an aqueous solution comprising a stabilizer (e.g. 2% PVOH aqueous solution) and homogenized for 4 min using an Ultra-Turrax T25 disperser at 24000 rpm to form an O/W emulsion. The emulsion was pH adjusted to 10 using NaOH solution (counted as the aqueous phase). This emulsion was then stirred at 500 rpm using a mechanical overhead stirrer and an aqueous solution of a polyaminoester (i.e compound 1) was slowly added over 1 hour. Once the addition was complete, the reaction temperature was gradually elevated to 70 °C over 1 h and was maintained at 70 °C for 2 h before being allowed to cool to room temperature.

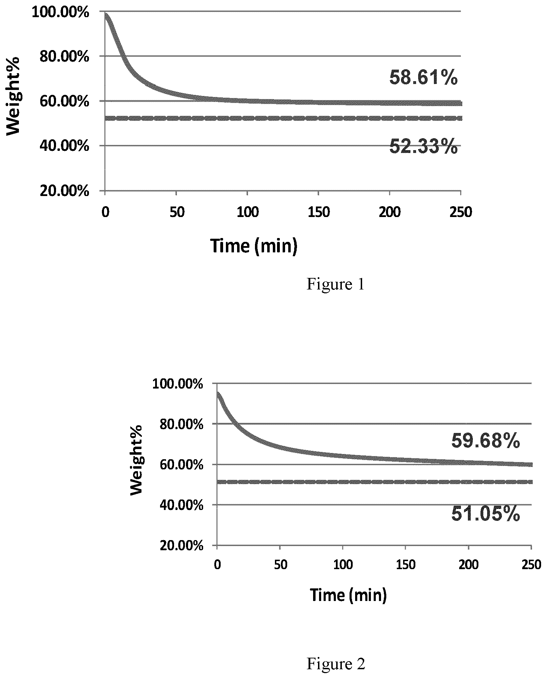

The average size of microcapsules A is around 8.5 µm. TGA measurement from 30°C to 50°C (5°C/min) and hold at 50°C for 250 min show that microcapsules retaine 100% of perfume oil after 250 min (see figure 1).

### Example 2

### Preparation of polyester (urea-urethane) microcapsules according to the invention

### Preparation of the polyaminoester crosslinker - oxybis(ethane-2,1-dyil) bis(2-aminoacetate)

The di-ammonium salt of L-glycine and diethylene glycol diester p-toluene sulfonate was synthesized as follows.

In a 500mL one-neck round bottom flask equipped with a Dean-Stark apparatus and a magnetic stirrer toluene (120mL) was added followed by α-amino acids, Glycine, (4.17g, 55mmol, 22eq.), p-toluenesulfonic acid monohydrate as catalyst (11.4g, 60mmol, 2.4eq.), and DEG (2.68g, 25mmol, 1.0eq.). The reaction mixture was refluxed for about 10hours (overnight, 130°C) until no more water was produced. After the reaction mixture was cooled to room temperature the toluene was removed under reduced pressure in the rotary evaporator. The resulting crude compound 3 was recrystallized from isopropyl/ethyl acetate (80/20). The melting point of the recrystallized product was: 118°C which is in the range reported (116°C-120°C) in the literature

To prepare the aqueous solution of compound 3, the di-ammonium salt of L-glycine and diethylene glycol diester p-toluene sulfonate is added into water followed by adding base (NaOH or Na₂CO₃) to deprotonate to obtain compound 3 (oxybis(ethane-2,1-dyil) bis(2-aminoacetate).

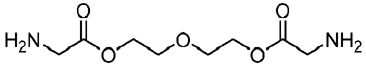

### Preparation of poly(ester urea) based microcapsules

**Table 3: Composition of capsules B according to the invention**

| **Ingredient** | **Percentage (w/w)** |
|---|---|
| Perfume Oil ¹⁾ | 31.88% |
| Takenate^{®} D-110N ²⁾ | 4.07% |
| Compound 3 | 9% |
| Water | 48.95% |
| Na₂CO₃ | 5.31% |
| PVOH | 0.79% |

| | |
|---|---|
| 1) Perfuming composition (perfume oil A) described in Table 2. 2) trimethylol propane adduct of xylylene diisocyanate; origin: Mitsui Chemicals, 75% polyisocyanate /25% ethyl acetate | |

The synthesis procedure is the same as in Example 1.

The average size of microcapsules B is around 10 µm. TGA measurement from 30°C to 50°C (5°C/min) and hold at 50°C for 250 min show that microcapsules retain 100% of perfume oil after 250 min (see figure 2).

### Example 3

### Olfactive performance of microcapsules according to the invention

Olfactive performance of PEU (polyester urea-based microcapsules) versus the free perfume oil A using the "swipe and sniff' method on a glass slide was determined.

Protocol: Two glass slides were presented to 29 panelists in a randomized order across panelists. The panelists were asked to smell the glass above the ¾" circle dot and rate the fragrance intensity in the sensory booths. Then they put a nitrile finger cot on their index finger and rubbed the glass above the dot from left to right 5 times and rated the fragrance intensity. After a 30 second break, they switched to a new finger cot and repeated the procedure for another sample, using the same rubbing force. Fragrance intensity was evaluated on a 0-10 continuous linear scale from "None" to "Very intense".

Sample preparation: The label with the three digit blind code was stuck to left side of the glass slide (Fisher brand plain microscope slides 25x75x1.0mm). A ¾" circle sticker was placed on the right bottom side of the glass slide to indicate the sample location. A 10uL of water slurry containing PEU capsules prepared according to Example 1 (0.3 wt% perfume oil A) or 0.3wt% free perfume oil A emulsion (90% water/10% ethanol) was pipetted onto the glass above the circle dot. The glass slides were dried at room temperature for approximately 20 hours before the evaluation.

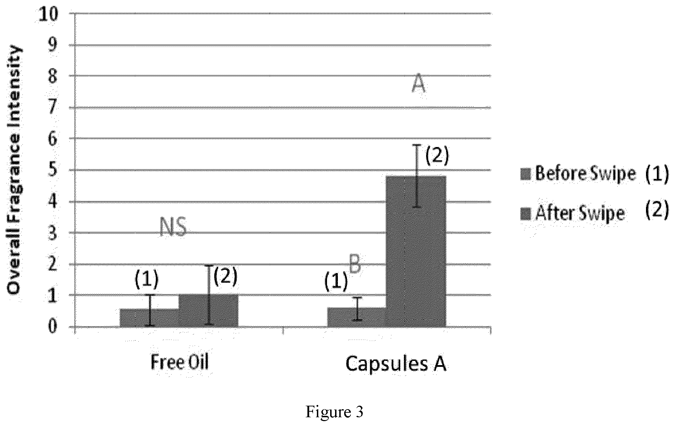

The sensory results indicated no significant difference between fragrance intensity of the free oil before and after swiping. For the microcapsules according to the invention, the fragrance intensity was significantly higher after swiping the slide than before swiping (see figure 3)
*: *different letter means that there is significant difference of fragrance intensity at 95% confidence level.*

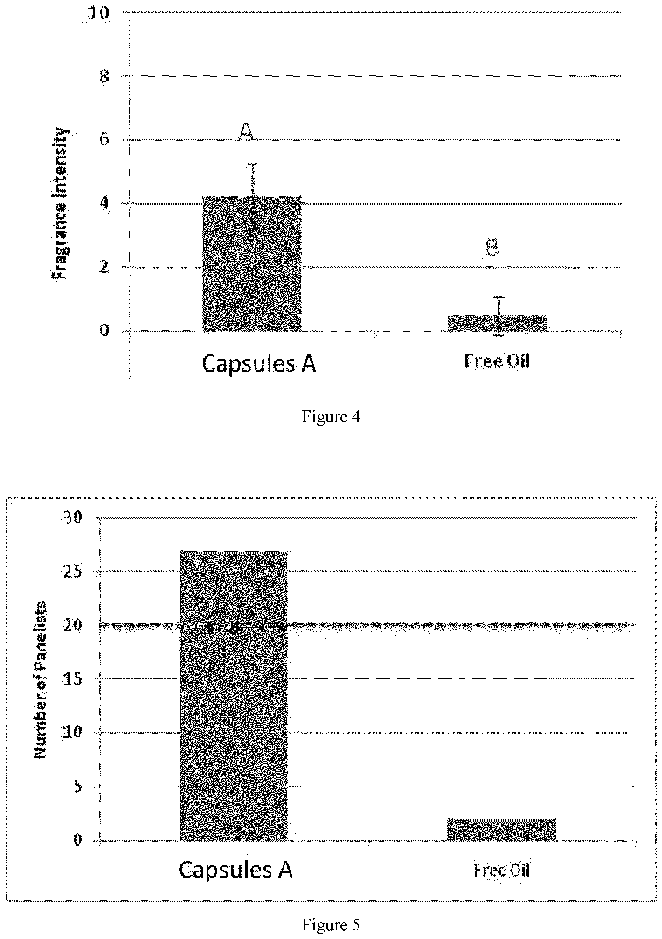

Additionally, when comparing the difference in fragrance intensity before and after swiping between microcapsules according to the invention and the free oil, the capsules show a significantly larger difference. This indicates that microcapsules according to the invention impart a significant boosting effect (see figure 4).

From statistic point of view, 27 out of 29 panelists chose the PEU capsules as stronger in overall fragrance intensity, which is significant at 95% confidence level (see figure 5).
*: *Dashed line indicates minimum number of selections to reach significance (α*=*0.05)*

### Example 4

### Shower gel comprising spray-dried poly(ester urea) based microcapsules according to the invention

Capsules slurry A and B (450mg) prepared respectively according to Examples 1 and 2 have been introduced in a shower gel base (10g) having the composition described in Table 4 (1.4% of perfume oil in the sample).

**Table 4: Shower gel base composition**

| **Ingredients** | **Amount (% wt)** | **Function** |
|---|---|---|
| WATER deionized | 52.40 | Solvent |
| Tetrasodium EDTA ¹⁾ | 0.10 | Chelating agent |
| Sodium Benzoate | 0.50 | Preservative |
| Propylene Glycol | 2.00 | Solvent |
| Sodium C12-C15 Pareth Sulfate ²⁾ | 35.00 | Surfactant |
| Cocamidopropyl Betaine³⁾ | 8.00 | Surfactant |
| Polyquaternium-7⁴⁾ | 0.20 | Conditioning agent |
| Citric Acid (40%) | 1.00 | pH adjuster |
| Sodium Chloride | 0.80 | Viscosity adjuster |

| | | |
|---|---|---|
| 1) EDETA B POWDER; trademark and origin: BASF 2) ZETESOL AO 328 U; trademark and origin: ZSCHIMMER & SCHWARZ 3) TEGO-BETAIN F 50; trademark and origin: GOLDSCHMIDT 4) MERQUAT 550; trademark and origin: LUBRIZOL | | |

Samples were stored at 42°C for 2 weeks - microscopic images demonstrated the stability of microcapsules according to the invention in the base.

### Example 5

### Spray-dried microcapsules according to the invention

Microcapsules C, D, E and F were prepared according the protocol defined in Example 1 by using perfume oil B having the following composition.

**Table 5: Microcapsules C, D, E and F**

| **Ingredient** | **Capsule C Weight%** | **Capsule D Weight%** | **Capsule E Weight%** | **Capsule F Weight%** |
|---|---|---|---|---|
| Perfume oil ¹⁾ | 31.05% | 26.93% | 35.80% | 32.92% |
| Takenate D110N ²⁾ | 3.96% | 3.44% | 4.59% | 4.18% |
| Compound 1 ³⁾ | 11.38% | 9.88% | 6.57% | 6.05% |
| Polyvinyl alcohol ⁴⁾ | 0.77% | 0.66% | 0.88% | 0.81% |
| Na₂CO₃ | 5.17% | 4.49% | 3.00% | 2.75% |
| Water | 47.67% | 54.60% | 49.15% | 53.28% |
| Total | 100.00% | 100.00% | 100.00% | 100.00% |

| | | | | |
|---|---|---|---|---|
| 1) see table 6 2) trimethylol propane adduct of xylylene diisocyanate; origin: Mitsui Chemicals, 75% polyisocyanate /25% ethyl acetate 3) Prepared according to Example 1 4) 2% Kurray C-506 | | | | |

**Table 6: Perfume oil B**

| **Ingredients** | **Concentration (Weight%)** |
|---|---|
| Ethyl 2-methyl-pentanoate | 0.25% |
| (Z)-3-hexen-1-ol acetate | 0.30% |
| 2,4-Dimethyl-3-cyclohexene-1-carbaldehyde | 1.25% |
| 4-methyl-2-(2-methyl-1-propen-1-yl)tetrahydro-2H-pyran | 0.40% |
| Hexyl Isobutyrate | 3.80% |
| Menthone | 0.50% |
| Menthol | 3.20% |
| Linallyl acetate | 10.20% |
| 1,1-dimethyl-2-phenylethyl acetate | 2.05% |
| Terpenyl acetate | 9.50% |
| Citronellyl acetate | 7.30% |
| Verdyl acetate | 3.00% |
| Nopyl acetate | 7.60% |
| Bétaionone | 0.50% |
| Verdyle Propionate | 4.20% |
| (+-)-methyl (3-oxo-4-pentylcyclopentyl)acetate | 11.00% |
| Aid hexylcinnamique | 13.50% |
| Tinogard ^{®}DA | 0.50% |
| Tinogard ^{®} TT DD | 0.20% |
| BHT | 0.10% |
| 1-(5,5-dimethyl-1-cyclohexen-1-yl)-4-penten-1-one | 0.10% |
| 2-(2-(4-methyl-3-cyclohexen-1-yl)propyl)cyclopentanone | 9.40% |
| Isopropyl Myristate | 11.15% |

The resulted suspension was then mixed well with maltodextrin 10DE, the carrier material, in water and spray dried (spray drying formulation shown in Table 7).

**Table 7: Spray Drying Formulation of microcapsules C, D, E and F**

| | C | D | E | F |
|---|---|---|---|---|
| Microcapsules slurry (wt%) | 65.8 | 68.9 | 62.5 | 64.5 |
| 10 DE Maltodextrin (wt%) | 34.2 | 31.1 | 37.5 | 35.5 |
| | 100 | 100 | 100 | 100 |

The feed solution was tuned by adding water to get proper viscosity and then the whole mixture was fed into a Buchi Spray Drier B-290. The inlet temperature was maintained at 180° C and the mixture was fed at a rate sufficient to maintain an outlet air temperature at 80° C. The spray dried white powder was obtained with a yield around 50%.

To confirm that there is no fragrance oil loss during microcapsule synthesis and spray drying process the total oil content of these 4 microcapsule samples was measured employing two different technologies (Low Field-NMR and GC-MS) as comparison to their theoretically calculated values (see table 8).

**Table 8: Total Oil Contents: Theoretical, LowField-NMR and GC-MS Results**

| | C | D |
|---|---|---|
| Theoretical | 29.74% | 29.74% |
| By LF-NMR | 29.75% | 30.23% |
| By GC-MS | 28.55% | 31.77% |

### Example 6

### Antiperspirant composition comprising spray-dried microcapsules according to the invention

An antiperspirant composition (stick) having the composition described in the table below is prepared.

| **Ingredient** | **Weight %** |
|---|---|
| DOW CORNING 345 Fluid (Cyclomethicone) | 55.000 |
| LANETTE 18 (Stearyl Alcohol) | 21.000 |
| TEGOSOFT PBE (PPG-14 Butyl Ether) | 2.000 |
| CUTINA HR (Hydrogenated Castor Oil) | 1.000 |
| SUMMIT AZP-908 (Aluminium Zirconium tetrachlorothdrex-Gly) | 20.000 |
| Perfume | 1.000 |
| Total | 100.00 |

The dosage of microcapsules C, D, E and F in the antiperspirant composition is determined to make the final perfume oil loading at 1%.

The sensory test was carried out using the "swipe and sniff" method on a paper blotter. AP stick weighed at 100 mg containing microcapsules encapsulating perfume oil was applied on each blotter. The blotters were evaluated by panellists based on the fragrance intensities before, and after rubbing, or water spraying (to mimic sweating after AP was applied to human body) followed by rubbing.

Once in the booth, 32 panelists were asked to evaluate a series of blotters. Each was blind-coded and there was a 30 second break in between evaluations.

Evaluate perfume intensity of AP with intact microcapsules on the blotter **(before rub):** In this first set, three blotters (one for each product) were presented in a random order. Panelists were asked to smell each blotter and rate the fragrance intensity.

Evaluate perfume intensity of AP with broken PEU microcapsules by rubbing on the blotter **(after rub).** In the second set, panelists were asked to evaluate three more paper blotters (one for each product). For each, they put a nitrile finger cot on their index finger, rubbed the blotter three times and rated the fragrance intensity. They changed the finger cot for each new sample.

Evaluate perfume intensity of wetted AP by spraying water and with broken microcapsules by rubbing on the blotter **(after spray&rub):** In the last set, panelists evaluated there more paper blotters, presented in a randomized order. First, they sprayed the blotter twice with DI water, then repeated the rubbing procedure. Fragrance intensity was evaluated on a 0-10 continuous linear scale from "None" to "Very intense".

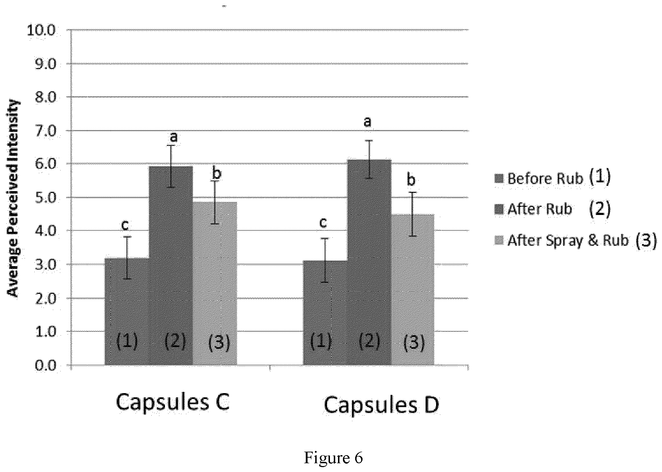

Results shown in figure 6 underline a significant boosting effect after rubbing compared with before rubbing.

## Claims

1. A process for the preparation of a poly(ester urea) based core-shell microcapsule slurry comprising the following steps:
a) dissolving at least one polyisocyanate having at least two isocyanate groups in a hydrophobic material, preferably a perfume, to form an oil phase;
b) preparing a dispersing phase comprising a stabilizer, wherein the dispersing phase is not miscible with the oil phase;
c) adding the oil phase obtained in step a) into the dispersing phase to form a two- phases dispersion;
d) performing a curing step to form core-shell microcapsules in the form of a slurry,
wherein a polyaminoester is added in step a) and/or in the two-phases dispersion of step c), and
wherein the polyaminoester is obtained by a reaction between a polyol and an amino-acid.

2. The process according to claim 1, wherein the polyol is chosen in the group consisting of glycerol, pentaerythritol, 1,1,1-tris(hydroxymethyl)ethane, 1,4-butanediol, diethylene glycol, and mixtures thereof.

3. The process according to claim 1, wherein the amino-acid is chosen in the group consisting of glycine, phenylalanine, alanine, valine, leucine, isoleucine and mixtures thereof.

4. The process according to anyone of the preceding claims, **characterized in that** the polyaminoester is selected from the group consisting of

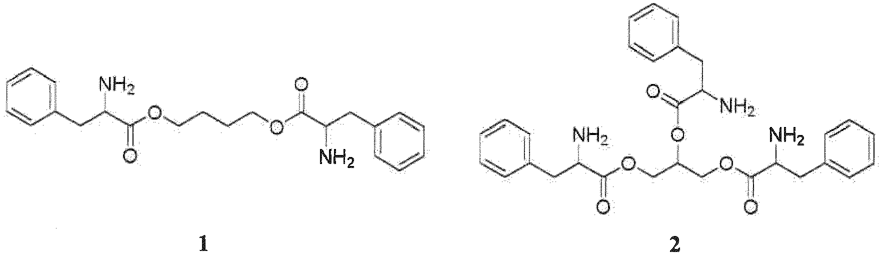

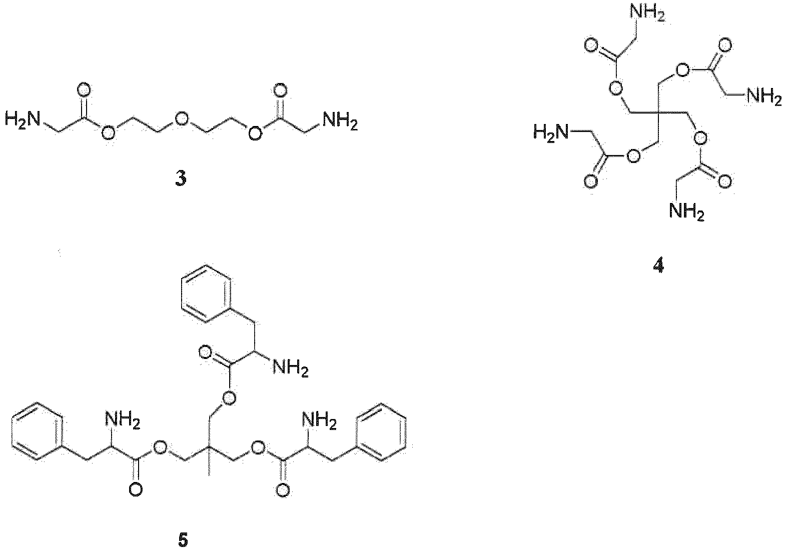

5. The process according to anyone of the preceding claims, wherein the polyaminoester is used in an amount of between 0.1 and 15%, preferably between 4 and 12%, these percentages being defined by weight relative to the total weight of the slurry.

6. The process according to anyone of the preceding claims, **characterized in that** the polyisocyanate is selected from the group consisting of a polyisocyanurate of toluene diisocyanate, a trimethylol propane-adduct of toluene diisocyanate and a trimethylol propane-adduct of xylylene diisocyanate, and mixtures thereof.

7. The process according to anyone of the preceding claims, **characterized in that** the stabilizer is chosen in the group consisting of gum Arabic, modified starch, polyvinyl alcohol, polyvinylpyrolidone (PVP), carboxymethylcellulose (CMC), anionic polysaccharides, acrylamide copolymer, inorganic particles, protein such as soy protein, rice protein, whey protein, white egg albumin, sodium caseinate, gelatin, bovine serum albumin, hydrolyzed soy protein, hydrolyzed sericin, pseudocollagen, silk protein, sericin powder, and mixtures thereof.

8. The process according to anyone of the preceding claims, **characterized in that** the polyisocyanate is used an amount of between 1 to 15%, preferably from 2 to 8% and more preferably from 2 to 6% by weight of the microcapsule slurry.

9. The process according to anyone of the preceding claims, **characterized in that** there is used an amount of between 20 and 50% of hydrophobic material, these percentages being defined by weight relative to the total weight of the microcapsule slurry.

10. The process according to anyone of the preceding claims, **characterized in that** the hydrophobic material comprises a perfume.

11. A poly(ester urea) based core-shell microcapsule comprising:
- an oil-based core comprising a hydrophobic material, preferably comprising a perfume oil, and
- a shell comprising a reaction product between at least one polyisocyanate having at least two isocyanate groups and a polyaminoester,
wherein the polyaminoester is obtained by a reaction between a polyol and an amino-acid.

12. A perfuming composition comprising
(i) Perfume microcapsule as defined in claim 11, wherein the hydrophobic material comprises a perfume,
(ii) at least one ingredient selected from the group consisting of a perfumery carrier, a perfumery co-ingredient and mixtures thereof , and
(iii) Optionally at least one perfumery adjuvant.

13. A liquid perfumed consumer product comprising:
a) from 2 to 65% by weight, relative to the total weight of the consumer product, of at least one surfactant;
b) water or a water-miscible hydrophilic organic solvent; and
c) the microcapsule as defined in claim 11 or the perfuming composition as defined in claim 12.

## Patentansprüche

1. Verfahren zur Herstellung einer auf einem Poly(ester-harnstoff) basierenden Kern-Hülle-Mikrokapsel-Suspension, das die folgenden Schritte umfasst:
a) Lösen mindestens eines Polyisocyanats, das mindestens zwei Isocyanatgruppen aufweist, in einem hydrophoben Material, vorzugsweise einem Parfüm, um eine Ölphase zu bilden;
b) Herstellen einer einen Stabilisator umfassenden dispergierenden Phase, wobei die dispergierende Phase nicht mit der Ölphase mischbar ist;
c) Zugeben der in Schritt a) erhaltenen Ölphase in die dispergierende Phase, um eine zweiphasige Dispersion zu bilden;
d) Durchführen eines Aushärtungsschritts, um Kern-Hülle-Mikrokapseln in der Form einer Suspension zu bilden,
wobei ein Polyaminoester in Schritt a) und/oder in die zweiphasige Dispersion aus Schritt c) zugegeben wird, und
wobei der Polyaminoester durch eine Umsetzung zwischen einem Polyol und einer Aminosäure erhalten wird.

2. Verfahren nach Anspruch 1, wobei das Polyol aus der Gruppe ausgewählt ist, die besteht aus Glycerin, Pentaerythritol, 1,1,1-Tris(hydroxymethyl)ethan, 1,4-Butandiol, Diethylenglykol und Mischungen davon.

3. Verfahren nach Anspruch 1, wobei die Aminosäure aus der Gruppe ausgewählt ist, die besteht aus Glycin, Phenylalanin, Alanin, Valin, Leucin, Isoleucin und Mischungen davon.

4. Verfahren nach einem der vorstehenden Ansprüche, **dadurch gekennzeichnet, dass** der Polyaminoester aus der Gruppe ausgewählt ist, die besteht aus

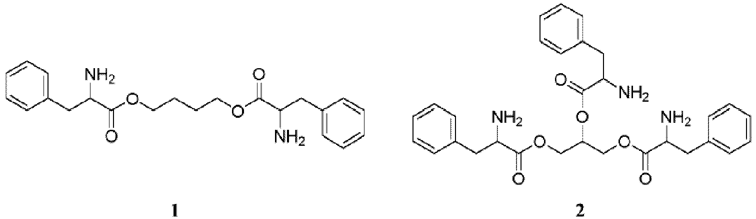

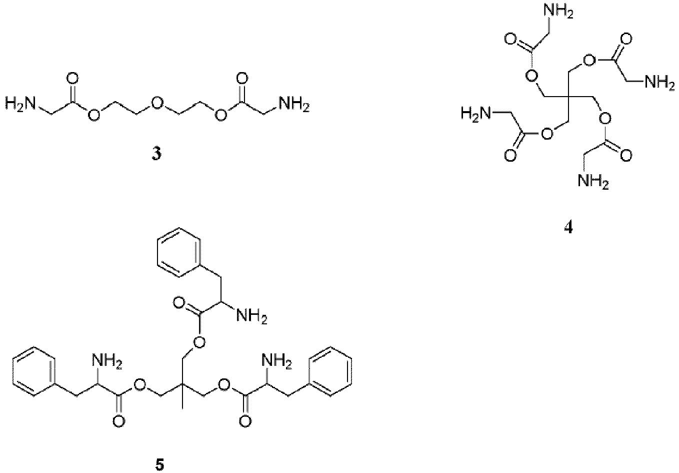

5. Verfahren nach einem der vorstehenden Ansprüche, wobei der Polyaminoester in einer Menge zwischen 0,1 und 15 %, vorzugsweise zwischen 4 und 12 %, verwendet wird, wobei diese Prozentanteile nach Gewicht bezogen auf das Gesamtgewicht der Suspension definiert sind.

6. Verfahren nach einem der vorstehenden Ansprüche, **dadurch gekennzeichnet, dass** das Polyisocyanat aus der Gruppe ausgewählt ist, die besteht aus einem Polyisocyanurat von Toluoldiisocyanat, einem Trimethylolpropan-Addukt von Toluoldiisocyanat und einem Trimethylolpropan-Addukt von Xylylendiisocyanat und Mischungen davon.

7. Verfahren nach einem der vorstehenden Ansprüche, **dadurch gekennzeichnet, dass** der Stabilisator aus der Gruppe ausgewählt ist, die besteht aus Gummiarabikum, modifizierter Stärke, Polyvinylalkohol, Polyvinylpyrrolidon (PVP), Carboxymethylcellulose (CMC), anionischen Polysacchariden, Acrylamid-Copolymer, anorganischen Teilchen, Protein, wie z. B. Sojaprotein, Reisprotein, Molkenprotein, Eiweißalbumin, Natriumcaseinat, Gelatine, bovinem Serumalbumin, hydrolysiertem Sojaprotein, hydrolysiertem Sericin, Pseudocollagen, Seidenprotein, Sericinpulver und Mischungen davon.

8. Verfahren nach einem der vorstehenden Ansprüche, **dadurch gekennzeichnet, dass** das Polyisocyanat in einer Menge zwischen 1 bis 15 %, bevorzugt von 2 bis 8 % und stärker bevorzugt von 2 bis 6 % nach Gewicht der Mikrokapsel-Suspension verwendet wird.

9. Verfahren nach einem der vorstehenden Ansprüche, **dadurch gekennzeichnet, dass** eine Menge zwischen 20 und 50 % hydrophoben Materials verwendet wird, wobei diese Prozentanteile nach Gewicht bezogen auf das Gesamtgewicht der Mikrokapsel-Suspension definiert sind.

10. Verfahren nach einem der vorstehenden Ansprüche, **dadurch gekennzeichnet, dass** das hydrophobe Material ein Parfüm umfasst.

11. Auf Poly(ester-harnstoff) basierende Kern-Hülle-Mikrokapsel, umfassend:
- einen auf Öl basierenden Kern, umfassend ein hydrophobes Material, vorzugsweise umfassend ein Parfümöl, und
- eine Hülle, umfassend ein Produkt einer Umsetzung zwischen mindestens einem Polyisocyanat, das mindestens zwei Isocyanatgruppen aufweist, und einem Polyaminoester,
wobei der Polyaminoester durch eine Umsetzung zwischen einem Polyol und einer Aminosäure erhalten wird.

12. Parfümierende Zusammensetzung, umfassend
(i) eine Parfümmikrokapsel gemäß Definition in Anspruch 11, wobei das hydrophobe Material ein Parfüm umfasst,
(ii) mindestens eine Ingredienz, die aus der Gruppe ausgewählt ist, die besteht aus einem Parfümträger, einer Parfüm-Koingredienz und Mischungen davon, und
(iii) wahlweise mindestens einen Parfümhilfsstoff.

13. Flüssiges parfümiertes Verbraucherprodukt, umfassend:
a) 2 bis 65 % nach Gewicht, bezogen auf das Gesamtgewicht des Verbraucherprodukts, von mindestens einem Tensid;
b) Wasser oder ein wassermischbares hydrophiles organisches Lösungsmittel; und
c) die Mikrokapsel gemäß Definition in Anspruch 11 oder die parfümierende Zusammensetzung gemäß Definition in Anspruch 12.

## Revendications

1. Procédé pour la préparation d'une suspension de microcapsules de type noyau-enveloppe à base de poly(ester-urée) comprenant les étapes suivantes :
a) dissolution d'au moins un polyisocyanate possédant au moins deux groupes isocyanates dans un matériau hydrophobe, de préférence un parfum, pour former une phase huileuse ;
b) préparation d'une phase de dispersion comprenant un stabilisant, dans lequel la phase de dispersion n'est pas miscible avec la phase huileuse ;
c) ajout de la phase huileuse obtenue dans l'étape a) dans la phase de dispersion pour former une dispersion biphasée ;
d) réalisation d'une étape de durcissement pour former des microcapsules de type noyau-enveloppe sous la forme d'une suspension,
dans lequel un polyaminoester est ajouté dans l'étape a) et/ou dans la dispersion biphasée de l'étape c), et
dans lequel le polyaminoester est obtenu par une réaction entre un polyol et un acide aminé.

2. Procédé selon la revendication 1, dans lequel le polyol est choisi dans le groupe constitué de : glycérol, pentaérythritol, 1,1,1-tris(hydroxyméthyl)éthane, 1,4-butanediol, diéthylène glycol et des mélanges de ceux-ci.

3. Procédé selon la revendication 1, dans lequel l' acide aminé est choisi dans le groupe constitué de : glycine, phénylalanine, alanine, valine, leucine, isoleucine et des mélanges de ceux-ci.

4. Procédé selon l'une quelconque des revendications précédentes, **caractérisé en ce que** le polyaminoester est choisi dans le groupe constitué de :

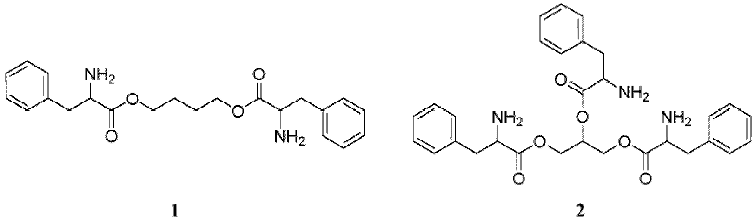

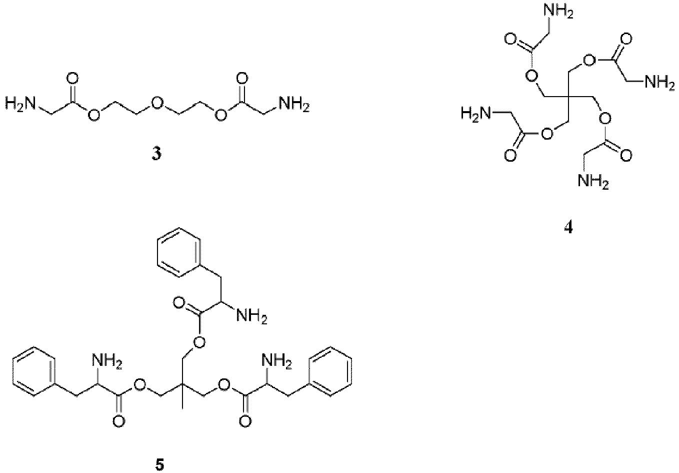

5. Procédé selon l'une quelconque des revendications précédentes, dans lequel le polyaminoester est utilisé en une quantité d'entre 0,1 et 15 %, de préférence entre 4 et 12 %, ces pourcentages étant définis en poids relativement au poids total de la suspension.

6. Procédé selon l'une quelconque des revendications précédentes, **caractérisé en ce que** le polyisocyanate est choisi dans le groupe constitué de : un polyisocyanurate de toluène diisocyanate, un adduit de triméthylol propane de toluène diisocyanate et un adduit de triméthylol propane de xylène diisocyanate et des mélanges de ceux-ci.

7. Procédé selon l'une quelconque des revendications précédentes, **caractérisé en ce que** le stabilisant est choisi dans le groupe constitué de : gomme arabique, amidon modifié, alcool polyvinylique, polyvinylpyrolidone (PVP), carboxyméthylcellulose (CMC), polysaccharides anioniques, copolymère d'acrylamide, particules inorganiques, protéine telle que protéine de soja, protéine de riz, protéine de petit-lait, albumine de blanc d'oeuf, caséinate de sodium, gélatine, albumine de sérum bovin, protéine de soja hydrolysée, séricine hydrolysée, pseudo-collagène, protéine de soie, poudre de séricine et des mélanges de ceux-ci.

8. Procédé selon l'une quelconque des revendications précédentes, **caractérisé en ce que** le polyisocyanate est utilisé en une quantité comprise entre 1 et 15%, de préférence de 2 à 8% et plus préférablement de 2 à 6% en poids de la suspension de microcapsules.

9. Procédé selon l'une quelconque des revendications précédentes, **caractérisé en ce qu'**il est utilisé une quantité comprise entre 20 et 50 % de matériau hydrophobe, ces pourcentages étant définis en poids relativement au poids total de la suspension de microcapsules.

10. Procédé selon l'une quelconque des revendications précédentes, **caractérisé en ce que** le matériau hydrophobe comprend un parfum.

11. Microcapsule de type noyau-enveloppe à base de poly(ester-urée) comprenant :
- un noyau à base d'huile comprenant un matériau hydrophobe, de préférence comprenant un parfum huileux, et
- une enveloppe comprenant un produit de réaction entre au moins un polyisocyanate possédant au moins deux groupes isocyanates et un polyaminoester,
le polyaminoester étant obtenu par une réaction entre un polyol et un acide aminé.

12. Composition parfumante comprenant :
(i) une microcapsule de parfum telle que définie dans la revendication 11, dans laquelle le matériau hydrophobe comprend un parfum,
(ii) au moins un ingrédient choisi dans le groupe constitué de : un support de parfumerie, un co-ingrédient de parfumerie et des mélanges de ceux-ci, et
(iii) optionnellement au moins un adjuvant de parfumerie.

13. Produit de consommation parfumé liquide comprenant :
a) de 2 à 65 % en poids, relativement au poids total du produit de consommation, d'au moins un tensioactif ;
b) de l'eau ou un solvant organique hydrophile miscible avec l'eau ; et
c) la microcapsule telle que définie dans la revendication 11 ou la composition parfumante telle que définie dans la revendication 12.
